# EUROPEAN PATENT SPECIFICATION

(11) **EP 1 915 151 B1**
(45) Date of publication and mention of the grant of the patent: **17.08.2011**
(21) Application number: 06739398.3
(22) Date of filing: 22.03.2006
(51) Int. Cl.: A61K 31/444, A61K 39/395, A61P 35/00

(54) **COMBINATIONS FOR THE TREATMENT OF CANCER COMPRISING ANTI-EGFR ANTIBODY AND VEGFR INHIBITORS**
ANTI-EGFR-ANTIKÖRPER UND VEGFR-INHIBITOREN UMFASSENDE KOMBINATIONEN FÜR DIE BEHANDLUNG VON KREBS
COMBINAISONS DESTINEES AU TRAITEMENT DE CANCER COMPRENANT ANTICORPS ANTI-EGFR INHIBITEURS VEGFR

(30) Priority: 22.03.2005 US 664381 P
(43) Date of publication of application: 30.04.2008
(73) Proprietor: Amgen, Inc, Thousand Oaks, California 91320-1799 (US)
(72) Inventor: CHANG, David, Calabasas, California 91302 (US)
(74) Representative: Grünecker, Kinkeldey, Stockmair & Schwanhäusser Anwaltssozietät
(86) International application number: PCT/US2006/010582
(87) International publication number: WO 2006/102504

(56) References cited:
- US-A1- 2003 225 106
- POLVERINO ANTHONY ET AL: "AMG 706, an Oral, Multikinase Inhibitor that Selectively Targets Vascular Endothelial Growth Factor, Platelet-Derived Growth Factor, and Kit Receptors, Potently Inhibits Angiogenesis and Induces Regression in Tumor Xenografts." CANCER RESEARCH. 1 SEP 2006, vol. 66, no. 17, 1 September 2006 (2006-09-01), pages 8715-8721, XP002399536 ISSN: 0008-5472
- LUWOR RODNEY B ET AL: "The antiepidermal growth factor receptor monoclonal antibody cetuximab/C225 reduces hypoxia-inducible factor-1 alpha, leading to transcriptional inhibition of vascular endothelial growth factor expression" ONCOGENE, vol. 24, no. 27, June 2005 (2005-06), pages 4433-4441, XP002399537 Published online 04.04.2005 ISSN: 0950-9232
- JUNG Y D ET AL: "Effects of combination anti-vascular endothelial growth factor receptor and anti-epidermal growth factor receptor therapies on the growth of gastric cancer in a nude mouse model" EUROPEAN JOURNAL OF CANCER, PERGAMON PRESS, OXFORD, GB, vol. 38, no. 8, May 2002 (2002-05), pages 1133-1140, XP004352362 ISSN: 0959-8049
- SHAHEEN R M ET AL: "INHIBITED GROWTH OF COLON CANCER CARCINOMATOSIS BY ANTIBODIES TO VASCULAR ENDOTHELIAL AND EPIDERMAL GROWTH FACTOR RECEPTORS" BRITISH JOURNAL OF CANCER, LONDON, GB, vol. 85, no. 4, 17 August 2001 (2001-08-17), pages 584-589, XP008052140 ISSN: 0007-0920
- HERBST, RS ET AL.: 40TH ASCO ANNUAL MEETING, NEW ORLEANS, LA (USA), 5 June 2004 (2004-06-05), - 8 June 2004 (2004-06-08) XP002399538 & HERBST ROY S ET AL: "Phase I/II trial evaluating the anti-vascular endothelial growth factor monoclonal antibody bevacizumab in combination with the HER-1/epidermal growth factor receptor tyrosine kinase inhibitor erlotinib for patients with recurrent non-small-cell lung cancer." JOURNAL OF CLINICAL ONCOLOGY : OFFICIAL JOURNAL OF THE AMERICAN SOCIETY OF CLINICAL ONCOLOGY. 10 APR 2005, vol. 23, no. 11, 10 April 2005 (2005-04-10), pages 2544-2555, XP002399538 ISSN: 0732-183X
- MORELLI MARIA PIA ET AL: "Anti-tumor activity of the combination of cetuximab, an anti-EGFR blocking monoclonal antibody and ZD6474, an inhibitor of VEGFR and EGFR tyrosine kinases" JOURNAL OF CELLULAR PHYSIOLOGY, vol. 208, no. 2, August 2006 (2006-08), pages 344-353, XP002399539 ISSN: 0021-9541
- TONRA JAMES R ET AL: "Synergistic antitumor effects of combined epidermal growth factor receptor and vascular endothelial growth factor receptor-2 targeted therapy." CLINICAL CANCER RESEARCH : AN OFFICIAL JOURNAL OF THE AMERICAN ASSOCIATION FOR CANCER RESEARCH. 1 APR 2006, vol. 12, no. 7 Pt 1, 1 April 2006 (2006-04-01), pages 2197-2207, XP002399540 ISSN: 1078-0432
- HUANG SHYHMIN ET AL: "Dual-agent molecular targeting of the epidermal growth factor receptor (EGFR): combining anti-EGFR antibody with tyrosine kinase inhibitor." CANCER RESEARCH. 1 AUG 2004, vol. 64, no. 15, 1 August 2004 (2004-08-01), pages 5355-5362, XP002399541 ISSN: 0008-5472

## Description

### Field of the Invention

This invention is in the field of pharmaceutical agents and specifically relates to the use of the anti-EGFR antibody panitumumab in combination with the VEGFR inhibitor AMG 706 for preparing a medicament for the management or treatment of non-small cell lung cancer, colon carcinoma or epidermoid carcinoma.

### Background

Protein kinases represent a large family of proteins which play central role in the regulation of a wide variety of cellular processes, maintaining control over cellular function. A partial list of such kinases includes ab1, Akt, bcr-ab1, Blk, Brk, Btk, c-kit, c-Met, c-src, c-frns, CDK1, CDK2, CDK3, CDK4, CDK5, CDK6, CDK7, CDK8, CDK9, CDK10, cRaf1, CSF1R, CSK, EGFR, ErbB2, ErbB3, ErbB4, Erk. Fak, fes, FGFR1, FGFR2, FGFR3, FGFR4, FGFR5, Fgr, flt-1, Fps, Frk, Fyn, Hck, IGF-1R INS-R, Jak, KDR, Lck, Lyn, MEK, p38, PDGFR, PIK, PKC, PYK2, ros, tie, tie2, TRK, Yes, and Zap70. Inhibition of such kinases has become an important therapeutic target.

Certain diseases are known to be associated with deregulated angiogenesis, for example ocular neovascularisation, such as retinopathies (including diabetic retinopathy), age-related macular degeneration, psoriasis, hemangioblastoma, bemangioma, arteriosclerosis, inflammatory disease, such as a rheumatoid or rheumatic inflammatory disease, especially arthritis (including rheumatoid arthritis), or other chronic inflammatory disorders, such as chronic asthma, arterial or post-transplamational atherosclerosis, endometriosis, and neoplastic diseases, for example so-called solid tumors and liquid tumors (such as leukemias).

At the center of the network regulating the growth and differentiation of the vascular system and its components, both during embryonic development and normal growth, and in a wide number of pathological anomalies and diseases, lies the angiogenic factor known as Vascular Endothelial Growth Factor" (VEGF; originally termed "Vascular Permeability Factor", VPF), along with its cellular receptors (see G. Breier et al., Trends in Cell Biology, 6;454-456 (1996)).

VEGF is a dimeric, disulfide-linked 46-kDa glycoprotein related to "Platelet-Derived Growth Factor" (PDGF); it is produced by normal cell lines and tumor cell lines; is an endothelial cen-specific mitogen; shows angiogenic activity in *in vivo* test systems (e.g. rabbit cornea); is chemotactic for endothelial cells and monocytes; and induces plasminogen activators in endothelial cells, which are involved in the proteolytic degradation of extracellular matrix during the formation of capillaries. A number of isoforms of VEGF are known, which show comparable biological activity, but differ in the type of cells that secrete them and in their heparin-binding capacity. In addition, there are other members of the VEGF family, such as "Placenta Growth Factor" (PIGF) and VEGF-C.

VEGF receptors (VEGFR) are transmembranous receptor tyrosine kinases. They are characterized by an extracellular domain with seven immunoglobulin-like domains and an intracellular tyrosine kinase domain. Various types of VEGF receptor are known, e.g. VEGFR-1 (also known as flt-1), VEGFR-2 (also known as KDR), and VEGFR-3.

A large number of human tumors, especially gliomas and carcinomas, express high levels of VEGF and its receptors. This has led to the hypothesis that the VEGF released by tumor cells stimulates the growth of blood capillaries and the proliferation of tumor endothelium in a paracrine manner and through the improved blood supply, accelerate tumor growth. Increased VEGF expression could explain the occurrence of cerebral edema in patients with glioma. Direct evidence of the role of VEGF as a tumor angiogenesis factor *in vivo* is shown in studies in which VEGF expression or VEGF activity was inhibited. This was achieved with anti-VEGF antibodies, with dominant-negative VEGFR-2 mutants which inhibited signal transduction, and with antisense-VEGF RNA techniques. All approaches led to a reduction in the growth of glioma cell lines or other tumor cell lines *in vivo* as a result of inhibited tumor angiogenesis.

Angiogenesis is regarded as an absolute prerequisite for tumors which grow beyond a diameter of about 1-2 mm; up to this limit, oxygen and nutrients may be supplied to the tumor cells by diffusion. Every tumor, regardless of its origin and its cause, is thus dependent on angiogenesis for its growth after it has reached a certain size.

Three principal mechanisms play an important part in the activity of angiogenesis inhibitors against tumors: 1) Inhibition of the growth of vessels, especially capillaries, into avascular resting tumors, with the result that there is no net tumor growth owing to the balance that is achieved between cell death and proliferation; 2) Prevention of the migration of tumor cells owing to the absence of blood flow to and from tumors; and 3) Inhibition of endothelial cell proliferation, thus avoiding the paracrine growth-stimulating effect exerted on the surrounding tissue by the endothelial cells which normally line the vessels. See R. Connell and J. Beebe, Exp. Opin. Ther. Patents, 11:77-114 (2001).

VEGF's are unique in that they are the only angiogenic growth factors known to contribute to vascular hyperpermeability and the formation of edema. Indeed, vascular hyperpermeability and edema that is associated with the expression or administration of many other growth factors appears to be mediated via VEGF production.

Inflammatory cytokines stimulate VEGF production. Hypoxia results in a marked upregulation of VEGF in numerous tissues, hence situations involving infarct, occlusion, ischemia, anemia, or circulatory impairment typically invoke VEGF/VPF-mediated responses. Vascular hyperpermeability, associated edema, altered transendothelial exchange and macromolecular extravasation, which is often accompanied by diapedesis, can result in excessive matrix deposition, aberrant stromal proliferation, fibrosis, etc. Hence, VEGF-mediated hyperpermeability can significantly contribute to disorders with these etiologic features. As such, regulators of angiogenesis have become an important therapeutic target. See Hicklin and Ellis, J. Clin. Oncology, 23:1011-1027 (2005).

Several observations implicate EGFr in supporting development and progression of human solid tumors. Signal, 2:2-35 (2001). Expression of EGFr has been shown to induce transformed properties in recipient cells. EGFr expression has been found to be up-regulated on many human tumors, including lung, colon, breast, prostate, gastric, brain, head and neck, ovarian and renal carcinoma, and the increase in receptor levels has been reported to be associated with a poor clinical prognosis. Mendelsohn, Cancer Cells, 7:359 (1989); Mendelsohn, Cancer Biology, 1:339-344 (1990); Modjtahedi and Dean, Int'l J. Oncology, 4:277-296 (1994). Modjtahedi and Dean, Int'l J. Oncology, 4:277-296 (1994). In many cases, the increased surface EGFr expression was accompanied by production of TGF or EGF by the tumor cells, suggesting the involvement of an autocrine growth control in the progression of these tumors. Both epidermal growth factor (EGF) and transforming growth factor-alpha (TGF-α) have been demonstrated to bind to EGF-r and to lead to cellular proliferation and tumor growth. These observations suggested that blocking the interaction between the growth factors and EGFr could result in arrest of tumor growth and possibly affect tumor survival.

Thus, certain groups have proposed that antibodies against EGF, TGF-a, and EGF-r may be useful in the therapy of tumors expressing EGF-r. Mendelsohn, Cancer Cells, 7:359 (1989); Mendelsohn, Cancer Biology, 1:339-344 (1990); Modjtahedi and Dean, Int'l J. Oncology, 4:277-296 (1994); Tosi et al., Int'l J. Cancer, 62:643-650 (1995). Indeed, it has been demonstrated that anti-EGF.r antibodies while blocking EGF and TGF-α binding to the receptor appear to inhibit tumor cell proliferation. At the same time, however, anti-EGF-r antibodies have not appeared to inhibit EGF and TGF-α independent cell growth. Modjtahedi and Dean, Int'l J. Oncology, 4:277-296 (1994). See also Cirdiello et al., Eur. J Cancer, 39:1348-1354 (2003).

MAbs specific to the human EGFr, capable of neutralizing EGF and TGFα binding to tumor cells and of inhibiting ligand-mediated cell proliferation in vitro, have been generated from mice and rats. Some of these antibodies, such as the mouse 108, 225 and 528 or the rat ICR16, ICR62 and ICR64 MAbs, were evaluated extensively for their ability to affect tumor growth in xenograft mouse models. Most of the anti-EGFr MAbs were efficacious in preventing tumor formation in athymic mice when administered together with the human tumor cells. When injected into mice bearing established human tumor xenografts, the mouse MAbs 225 and 528 caused partial tumor regression and required the co-administration of chemotherapeutic agents, such as doxorubicin or cisplatin, for eradication of the tumors. A chimeric version of the 225 MAb (C225), in which the mouse antibody variable regions are linked to human constant regions, exhibited an improved *in vivo* anti-tumor activity but only at high doses. The rat ICR16, ICR62, and ICR64 antibodies caused regression of established tumors but not their complete eradication. These results established EGFr as a promising target for antibody therapy against EGFr-expressing solid tumors and led to human clinical trials with the C225 MAb in multiple human solid cancers. Therefore, anti-EGFr antibody therapy can be fully evaluated with the availability of a fully human anti-EGFr antibody that exhibits therapeutic efficacy on EGFr-expressing tumors and that can be administered repeatedly to all appropriate patient populations.

A number of murine and rat monoclonal antibodies against EGF-r have been developed and tested for their ability to inhibit the growth of tumor cells *in vitro* and *in vivo.* Modjtahedi and Dean, Int'l J. Oncology, 4:277-296 (1994). The murine antibody, designated 225, upon which the C225 antibody is based, was developed by University of California and Rorer. See U.S. Patent No. 4,943,533 and European Patent No. 359,282. C225 was demonstrated to inhibit EGF-mediated tumor cell growth *in vitro* and inhibit human tumor formation *in vivo* in nude mice. The antibody, moreover, appeared to act in synergy with certain chemotherapeutic agents to eradicate human tumors *in vivo* in xenograft mouse models. Modjtahedi and Dean, Int'l J. Oncology, 4:277-296 (1994). ImClone is marketing the anti-EGF-r antibody C225 now designated Erbitux (cetuximab).

Yang et al. describe the effect of a fully human monoclonal antibody to EGFr on tumors. Cancer Res., 59:1236-1243 (1999).

Combinations of antibodies targeting VEGFR and EGFR for the treatment of colon cancer were described by Shaheen et al., Brit. J. of Cancer, 85:584-589 (2001). A combination of a EGFR inhibitor and gemcitabine for the treatment of pancreatic carcinomas was described by Bruns et al., Cancer Res., 60:2926-2935 (2000) and Clin. Cancer Res., 6:1936-1948 (2000). A combination of Iressa and inhibitors of PKAI for the treatment of colon and breast cancer was described by Tortora et al., Clin. Cancer Res., 9:866-871 (2003). A combination of paclitaxel and Iressa for the treatment of a variety of cancers was described by Ciardiello et al., Clin. Cancer Res., 7:1459-1465 (2001). A combination of paclitaxel and EGFR antibody C225 for the treatment of bladder transitional cell carcinoma was described by Inoue et al., Clin. Cancer Res., 6:4874-4884 (2000) and Clin. Cancer Res., 6:2635-2643 (2000). Herbst et al. (J. Clin. Oncol., 23(11):2544-2555 (2005)) describe data on a VEGF antibody and EGFR inhibitor erlotinib in lung cancer. A combination of antibodies targeting VEGFR and EGFR for the treatment of gastric cancer were described by Jung et al., Eur. J Cancer, 38:1133-1140 (2002).

It is now found that some combinations of a VEGF pathway inhibitor and an antibody that inhibits the EGFR pathway provides better results than one or the other inhibitor used alone.

### Description of the Drawings

**Figure 1** shows the combination of VEGFR inhibitor AMG 706 and anti-EGFR antibody panitumumab are most effective in the treatment of A431 human epidermoid carcinoma cells.
**Figure 2** shows the combination of VEGFR inhibitor AMG 706 and anti-EGFR antibody panitumumab are most effective in the treatment of HT29 human colon carcinoma cells.
**Figure 3** shows the combination of VEGFR inhibitor AMG 706 and anti-EGFR antibody panitumumab are most effective in the treatment of HT29 human colon carcinoma cells.
**Figure 4** shows the combination of VEGFR inhibitor AMG 706 and anti-EGFR antibody panitumumab are most effective in the treatment of CALU6 human non-small cell lung cancer cells.
Figure 5 shows the combination of VEGFR inhibitor A and Erbitux are effective in the treatment of CALU6 human non-small cell lung cancer cells.

### DETAILED DESCRIPTION OF THE INVENTION

1. Use of an anti-EGFR fully human antibody in combination with a VEGFR inhibitor, wherein the anti-EGFR fully human antibody is panitumumab and the VEGFR inhibitor is N-(2,3-dihydro-3,3-dimethyl-1H-indol-6-yl)-2-[(4-pyridinylmethyl)amino]-3-pyridinecarboxamide, for manufacture of a medicament for the management or treatment of non-small cell lung cancer, colon carcinoma and epidermoid carcinoma in a subject
2. The use of Item 1, wherein the medicament is for the management or treatment of non-small cell lung cancer.
3. The use of Item 1, wherein the medicament is for the management of treatment of colon carci noma.
4. The use of Item 1, wherein the medicament is for the management of treatment of epl dermold carcinoma.
5. The use of any of Items 1-4, wherein the anti-EGFR fully human antibody is administered in a dose of about 2mg/kg to about 3 mg/kg per week, about 5 mg/kg to about 7 mg/kg every two weeks or about 8 mg/kg to about 10 mg/kg every three weeks.
6. The use of any of Items 1-4, wherein the VEGFR inhibitor is administered in a dose of about 25 mg to about 125 mg.
7. The use of any of Items 1-4, wherein the VEGFR inhibitor is administered in a dose of about 75 mg twice a day.
8. The use of any of Items 1-4, wherein the VEGFR inhibitor is administered in a dose of about 100 mg twice a day.
9. The use of any of Items 1-4, wherein the VEGFR inhibitor is administered in a dose of about 125 mg once a day.
10. A combination of an anti-EGFR fully human antibody and a VEGFR inhibitor, wherein the anti-EGFR fully human antibody is panitumumab and the VEGFR inhibitor is N-(2,3-dihydro-3,3-dimethyl-1H-indol-6-yl)-2-[(4-pyridinylmethyl)amino]-3-pyridinecarboxamide, for use in the management or treatment of cancer in a subject, wherein the cancer is selected from non-small cell lung cancer, colon carcinoma and epidermoid carcinoma.
11. The combination of Item 10, wherein the anti-EGFR fully human antibody is administered in a dose of about 2mg/kg to about 3 mg/kg per week, about 5 mg/kg to about 7 mg/kg every two weeks or about 8 mg/kg to about 10 mg/kg every three weeks.
12. The combination of Item 10, wherein the VEGFR inhibitor is administered in a dose of about 25 mg to about 125 mg.
13. The combination of Item 10, wherein the VEGFR inhibitor is administered in a dose of about 75 mg twice a day.
14. The combination of Item 10, wherein the VEGFR inhibitor is administered in a dose of about 100 mg twice a day.
15. The combination of Item 10, wherein the VEGFR inhibitor is administered in a dose of about 125 mg once a day.

"Adjuvant chemotherapy" is defined as the continued treatment after either intensive cycles of chemotherapy and/or radiation, or alternatively after surgery to remove tumors. Alternatively the term describes the use of drugs as additional treatment for patients with cancers that are thought to have spread outside their original sites. Neo-adjuvant therapy is defined as intensive cycles of chemotherapy and/or radiation given to reduce the size of tumor before a definitive surgery. Such adjuvant or neo-adjuvant chemotherapy +/radiation relates to the treatment of neoplasea including, but not limited to: carcinoma of the breast, colon, lung, and head and neck.

The invention describes combinations with a VEGFR inhibitor of the formula 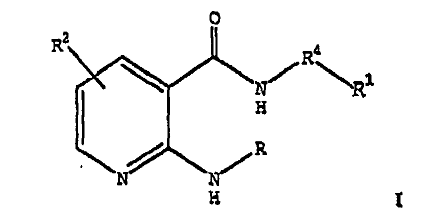 wherein R is selected from unsubstituted or substituted 9- or 10-membered fused nitrogen-containing heteroaryl,
wherein R is substituted with one or more substituents selected from halo, amino,
hydroxy, C₁₋₆-alkyl, C₁₋₆haloalkyl, C₁₋₆-alkoxy, optionally substituted heterocyclylalkoxy, C₁₋₆-alkylamino-C₂₋₄-alkynyl, C₁₋₆-alkylamino-C₁₋₆-alkoxy, C₁₋₆-alkylamino-C₁₋₆-alkoxy-C₁₋₆-alkoxy, and optionally substituted heterocyclyl-C₂₋₄-alkynyl;
wherein R¹ is selected from unsubstituted or substituted
aryl,
cycloalkyl,
5-6 membered heteroaryl and
9-10 membered bicyclic and 13-14 membered tricyclic heterocyclyl,
wherein substituted R¹ is substituted with one or more substituents selected from halo,
C₁₋₆-alkyl, optionally substituted C₁₋₆-cycloalkyl, optionally substituted phenyl, optionally substituted phenyl-C₁₋C₄-alkylenyl, C₁₋₂-haloalkoxy, optionally substituted phenyloxy, optionally substituted 4-6 membered heteracyclyl-C₁₋C₄-alkyl, optionally substituted 4-6 membered heterocyclyl-C₂₋C₄-alkenyl, optionally substituted 4-6 membered heterocyclyl, optionally substituted 4-6 membered heterocyclyloxy, optionally substituted 4-6 membered beterocyclyl-C₁₋₄-alkoxy, optionally substituted 4-6 membered heterocyclylsulfonyl, optionally substituted 4-6 membered heterocyclylamino, optionally substituted 4-6 membered heterocyclylcarbonyl, optionally substituted 4-6 membered heterocyclyl-C₁₋₄-alkylcarbonyl, C₁₋₂-haloalkyl, C₁₋₄-aminoalkyl, nitro, amino, hydroxy, cyano, aminosulfonyl, C₁₋₂-alkylsulfonyl, halosulfonyl, C₁₋₄-alkylcarbonyl, C₁₋₃-alkylamino-C₁₋₃-alkyl, C₁₋₃-alkylamino-C₁₋₃-alkoxy, C₁₋₃-alkylamino-C₁₋₃-alkoxy-C₁₋₃-alkoxy, C₁₋₄-alkoxycarbonyl, C₁₋₄-alkoxycarbonylamino-C₁₋₄-alkyl, C₁₋₄-hydroxyalkyl, 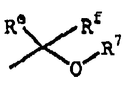 and C₁₋₄-alkoxy;
wherein R² is one or more substituents independently selected from H, halo, hydroxy, amino, C₁₋₆-alkyl, C₁₋₆-haloalkyl, C₁₋₆-alkoxy, C₁₋₂-alkylamino, aminosulfonyl, C₃₋₆-cyoloalkyl, cyano, C₁₋₂-hydroxyalkyl, nitro, C₂₋₃-alkenyl, C₂₋₃-alkynyl, C₁₋₆-haloalkoxy, C₁₋₆-carboxyalkyl, 4-6-membered heterocyclyl-C₁₋₆-alkylamino, unsubstituted or substituted phenyl and unsubstituted or substituted 4-6 membered heterocyclyl;
wherein R⁴ is selected from a direct bond, C₁₋₄-alkyl, and 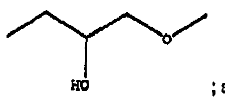 and
wherein R^{e} and R^{f} are independently selected from H and C₁₋₂-haloalkyl; and
wherein R⁷ is selected from H, C₁₋₃-alkyl, optionally substituted phenyl, optionally substituted phenyl-C₁₋₃-alkyl, 4-6 membered heterocyclyl, optionally substituted 4-6 membered heterocyclyl-C₁₋C₃-alkyl, C₁₋₃-alkoxy-C₁₋₂-alkyl and C₁₋₃-alkoxy-C₁₋₃-alkoxy-C₁₋₃-alkyl;
and pharmaceutically acceptable derivatives thereof.

The invention describes combinations with a VEGFR inhibitor of the formula 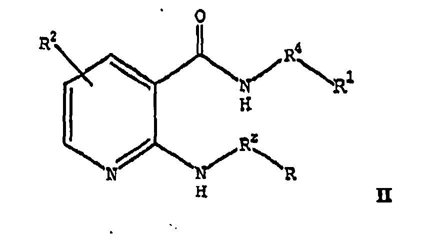 wherein R is selected from
a) unsubstituted or substituted 5- or 6-membered nitrogen-containing heteroaryl, and
b) unsubstituted or substituted 9- or 10-membered fused heteroaryl,
   where substituted R is substituted with one or more substituents selected from halo, amino, hydroxy, C₁₋₆-alkyl, C₁₋₆-haloalkyl, C₁₋₆-alkoxy, optionally substituted heterocyclyl-C₁₋₆-alkoxy, optionally substituted heterocyclyl-C₁₋₆-alkylamino, optionally substituted heterocyclyl-C₁₋₆-alkyl, C₁₋₆-alkylamino-C₂₋₄-alkynyl, C₁₋₆-alkylamino-C₁₋₆-alkoxy, C₁₋₆-alkylamino-C₁₋₆-alkoxy-C₁₋₆-alkoxy, and optionally substituted heterocyclyl-C₂₋₄-alkynyl;
   wherein R¹ is a ring selected from unsubstituted or substituted 4-6 membered saturated or partially un-saturated monocyclic heterocyclyl, 9-10 membered saturated or partially un-saturated bicyclic heterocyclyl, and 13-14 membered saturated or partially un-saturated tricyclic heterocyclyl,
   wherein substituted R¹ is substituted with one or more substituents selected from halo, C₁₋₆-alkyl, optionally substituted C₃₋₆-cycloalkyl, optionally substituted phenyl, optionally substituted phenyl-C₁₋C₄-alkylenyl, C₁₋₂-haloalkoxy, optionally substituted 4-6 membered heterocyclyl-C₁₋C₄-alkyl, optionally substituted 4-6 membered heterocyclyl-C₂₋C₄-alkenyl, optionally substituted 4-6 membered heterocyclyl, optionally substituted phenyloxy, optionally substituted 4-6 membered heterocyclyloxy, optionally substituted 4-6 membered heterocyclyl-C₁₋C₄-alkoxy, optionally substituted 4-6 membered heterocyclylsulfonyl, optionally substituted 4-6 membered heterocyclylamino, optionally substituted 4-6 membered heterocyclylcarbonyl, optionally substituted 5-6 membered heterocyclyl-C₁₋₄-alkylcarbonyl, C₁₋₂-haloalkyl, C₁₋₄-aminoalkyl, nitro, amino, hydroxy, oxo, cyano, aminosulfonyl, C₁₋₂-alkylsulfonyl, halosulfonyl, C₁₋₄-alkylcarbonyl, C₁₋₃-alkylamino-C₁₋₃-alkyl, C₁₋₃-alkylamino-C₁₋₃-alkoxy, C₁₋₃-alkylamino-C₁₋₃-alkoxy-C₁₋₃-alkoxy, C₁₋₄-alkoxycarbonyl, C₁₋₄-alkoxycarbonylamino-C₁₋₄-alkyl, C₁₋₄-hydroxyalkyl, 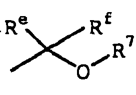 and C₁₋₄-alkoxy;
   wherein R² is one or more substituents independently selected from H, halo, hydroxy, amino, C₁₋₆-alkyl, C₁₋₆-haloalkyl, C₁₋₆-alkoxy, C₁₋₂-alkylamino, aminosulfonyl, C₃₋₆-cycloalkyl, cyano, C₁₋₂-hydroxyalkyl, nitro, C₂₋₃-alkenyl, C₂₋₃-alkynyl, C₁₋₆-haloalkoxy, C₁₋₆₋caboxyalkyl, 5-6-membered heterocyclyl-C₁₋₆-alkylamino, unsubstituted or substituted phenyl and unsubstituted or substituted 5-6 membered hcterocyclyl;
   wherein R⁴ is selected from a direct bond, C₁₋₄-alkyl, and 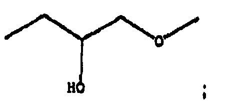 wherein R⁵ is selected from ₁₋₂-alkyl, C₂₋₆-branched alkyl, C₂₋₄-branched haloalkyl, amino-C₁₋₄-alkyl and C₁₋₂-alkylamino-C₁₋₂-alkyl:
   wherein R^{e} snd R^{f} are independently selected from H and C₁₋₂-haloalkyl; and
   wherein R⁷ is selected from H, C₁₋₃-alkyl, optionally substituted phenyl, optionally substituted phenyl-C₁₋₃-alkyl, optionally substituted 4-6 membered heterocyclyl, optionally substituted 4-6 membered heterocyclyl-C₁₋C₃-alkyl, C₁₋₃-alkoxy-C₁₋₂-alkyl and C₁₋₃-alkoxy-C₁₋₃-alkoxy-C₁₋₃-alkyl;
   and pharmaceutically acceptable isomers and derivatives thereof.

The invention describes combinations with a VEGFR inhibitor of the formula 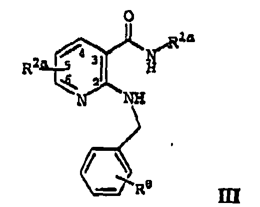 wherein R^{2a} is one or more substituents independently selected from H, halo, hydroxy, amino, C₁₋₆-alkyl, C₁₋₆-haloalkyl, C₁₋₆-alkoxy, C₁₋₂-alkylamino, aminosulfonyl, C₃₋₆-cycloalkyl, cyano, oxo, C₁₋₂-hydroxyalkyl, nitro, C₂₋₃-alkenyl, C₂₋₃-alkynyl, C₁₋₆-haloalkoxy, C₁₋₆-carboxyalkyl, 5-6-membered heterocyclyl-C₁₋₆-alkylamino, unsubstituted or substituted phenyl and unsubstituted or substituted 5-6 membered heterocyclyl;
wherein R¹⁰ is selected from unsubstituted or substituted phenyl, and
9-10 membered bicyclic and 13-14 membered tricyclic unsaturated or partially unsaturated heterocyclyl,
wherein substituted R^{1a} is optionally substituted with one or more substituents selected from halo, C₁₋₆-alkyl, optionally substituted C₃₋₆-cycloalkyl, optionally substituted phenyl, optionally substituted phenyl-C₁₋C₄-alkyl, C₁₋₂-haloalkoxy, optionally substituted phenyloxy, optionally substituted 4-6 membered heterocyclyl-C₁₋C₄-alkyl, optionally substituted 4-6 membered heterocyclyl-C₁₋C₄-alkenyl, optionally substituted 5-6 membered heterocyclyl, optionally substituted 4-6 membered heterocyclyloxy, optionally substituted 4-6 membered heterocyclyl-C₁₋C₄-alkoxy, optionally substituted 5-6 membered heterocyclylsulfonyl, optionally substituted 5-6 membered heterocyclylamino, optionally substituted 5-6 membered heterocyclylcarbonyl, optionally substituted 5-6 membered heterocyclylcarbonyl-C₁₋₄-alkyl, optionally substituted 5-6 membered heterocyclyl-C₁₋₄-alkylcarbonyl, C₁₋₄-haloalkyl, C₁₋₄-aminoalkyl, nitro, amino, hydroxy, oxo, cyano, aminosulfonyl, C₁₋₂-alkylsulfonyl, halosulfonyl, C₁₋₄-alkylcarbonyl, amino-C₁₋₄-alkylcarbonyl, C₁₋₄-alkylamino-C₁₋₄-alkylcarbonyl, C₁₋₃-alkylamino-C₁₋₃-alkyl, C₁₋₃-alkylamino-C₁₋₃-alkoxy, C₁₋₃-alkylamino-C₁₋₃-alkoxy-C₁₋₃-alkoxy, C₁₋₄-alkoxycarbonyl, C₁₋₄-alkoxycarbonylamino-C₁₋₄-alkyl, C₁₋₄-hydroxyalkyl, 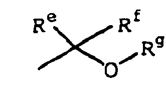 and C₁₋₄-alkoxy;
wherein R^{e} and R^{f} are independently selected from H and C₁₋₂-haloalkyl;
wherein R⁷ is selected from H, C₁₋₃-alkyl, optionally substituted phenyl-C₁₋₃-alkyl, 4-6 membered heterocyclyl, and optionally substituted 4-6 membered heterocyclyl-C₁₋C₃-alkyl;
wherein R⁸ is selected from H, C₁₋₃-alkyl, optionally substituted phenyl-C₁₋₃-alkyl, 4-6 membered heterocyclyl, and optionally substituted 4-6 membered heterocyclyl-C₁₋C₃-alkyl, C₁₋₃-alkoxy-C₁₋₂-alkyl and C₁₋₃-alkoxy-C₁₋₃-alkoxy-C₁₋₃-alkyl; and
wherein R⁸ is one or more substituents independently selected from H, halo, amino, hydroxy, C₁₋₆-alkyl, C₁₋₆-haloalkyl, C₁₋₆-alkoxy, C₁₋₆-haloalkoxy, C₁₋₆-aminoalkyl, C₁₋₆-hydroxyalkyl, optionally substituted phenyl, optionally substituted heterocyclyl, optionally substituted heterocyclyl-C₁₋₆-alkoxy, aminosulfonyl, C₃₋₆-cycloalkyl, C₁₋₆-alkylamino, C₁₋₆-alkylamino-C₁₋₆-alkyl, optionally substituted heterocyclyl-C₁₋₆-alkylamino, optionally substituted heterocyclyl-C₁₋₆-alkyl, C₁₋₆-alkylamino-C₂₋₄-alkynyl, C₁₋₆-alkylamino-C₁₋₆-alkoxy, C₁₋₆-alkylamino-C₁₋₆-alkoxy-C₁₋₆-alkoxy, and optionally substituted heterocyclyl-C₂₋₄-alkynyl;
and pharmaceutically acceptable isomers and derivatives thereof.

The invention describes combinations with a VEGFR inhibitor of the formula 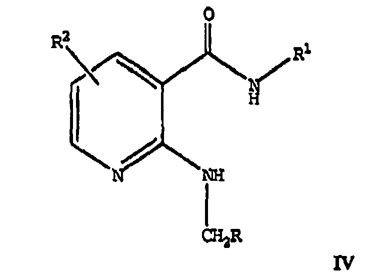 wherein R is selected from
a) unsubstituted or substituted 5- or 6-membered rings-selected from 4-pyridyl, 2-pyridyl, 4-pyrimidinyl, and tetrahydro-2H-pyran-yl, and
b) unsubstituted or substituted 9- or 10-membered fused rings selected from 4-quinolyl, 6-quinolyl, 2,3-dihydro-5-benzofuryl, 5-benzoxazolyl, 1H-pyrrolo[2,3-b]pyridin-4-yl, and 2,3-dihydro-1H-pyrrolo[2,3-b]pyridin-4-yl,
   where substituted R is substituted with one or more substituents selected from
   methylamino-, amino, methoxy, methylaminocarbonyl, morpholino, and trifluoromethoxy;
   wherein R¹ is 4,4-dimethyl-3,4-dihydro-2-oxo-1H-quinolinyl;
   or wherein R¹ is 4,4-dimethyl-1,2,3,4-tetrahydro-1H-quinolinyl;
   or wherein R¹ is 4,4-dimethyl-3,4-dihydro-2-oxo-1H-[1,8]-naphthyridinyl;
   or wherein R¹ is 3,3-dimethyl-2,3-dihydro-1H-indolyl optionally substituted with
   a substituent selected from pyrrolidin-1-yl-carbonyl, methylcarbonyl, and methylsulfonyl;
   or wherein R¹ is 4,4-dimethyl-1,2,3,4-tetrahydro-1H-isoquinolinyl;
   or wherein R¹ is 2-oxo-3,3-bis(trifluoromethyl)-2,3-dihydro-1H-indol-6-yl;
   or wherein R¹ is 1',2'-dihydro-spiro[cyclopropane-1,3'-[3H]indol]-6'-yl; and
   wherein R² is H;
   and pharmaceutically acceptable isomers and derivatives thereof.
   The invention also relates to co-therapies with VEGFR inhibitors including N-(4-chlorophenyl)-4-(4-pyridinylmethyl)-1-phthalazinamime;
   N-(4-(1,1 -dimethylethyl)phenyl)-2-((4-pyridinylmethyl)amino)-3-pyridinecarboxamide
   (VEGF Inhibitor A);
   4-[4-[[[[4-chloro-3-(trifluoromethyl)phenyl]amino]carbonyl]amino]phenoxy]-N-methyl-2-pyridinecarboxamide;
   N-[2-(diethylamino)ethyl]-5-[(5-fluoro-1,2-dihydro-2-oxo-3H-indol-3-ylidene)methyl]-2,4-dimethyl-1H-pyrrole-3-carboxamide;
   3-[(4-bromo-2,6-difluorophenyl)methoxy]-5-[[[[4-(1-pyrrolidinyl)butyl]amino]carbonyl] amino]-4-isothiazolecarboxamide;
   N-(4-bromo-2-fluorophenyl)-6-methoxy-7-[(1-methyl-4-piperidinyl)methoxy]-4-quinazolinamine;
   3-[5,6,7,13-tetrahydro-9-[(1-methylethoxy)methyl]-5-oxo-12H-indeno[2,1-a]pyrrolo[3,4-c]carbazol-12-yl]propyl ester N,N-dimethyl-glycine;
   N-[5-[[[5-(1,1-dimethylethyl)-2-oxazolyl]methyl]thio]-2-thiazolyl]-4-piperidinecarboxamide;
   *N*-[3-chloro-4-[(3-fluorophenyl)methoxy]phenyl]-6-[5-[[[2-(methylsulfonyl)ethyl]amino] methyl]-2-furanyl]-4-quinazolinamine
   4-[(4-Methyl-1-piperazinyl)methyl]-N-[4-methyl-3-[[4-(3-pyridinyl)-2-pyrimidinyl] amino]-phenyl]benzamide
   *N*-(3-chloro-4-fluorophenyl)-1-methoxy-6-[3-(4-morpholinyl)propoxy]-4-quinazolinamine
   *N*-(3-ethynylphenyl)-6,7-bis(2-methoxyethoxy)-4-quinazolinamine
   N-(3-((((2R)-1-methyl-2-pyrrolidinyl)methyl)oxy)-5-(trifluoromethyl)phenyl)-2-((3-(1,3-oxazol-5-yl)phenyl)amino)-3-pyridinecarboxamide;
   2-(((4-fluorophenyl)methyl)amino)-N-(3-((((2R)-1-methyl-2-pyrrolidinyl)methyl)oxy)-5-(tri fluoromethyl)phenyl)-3-pyridinecarboxamide;
   N-[3-(Azetidin-3-ylmethoxy)-5-trifluoromethyl-phenyl]-2-(4-fluoro-benzylamino)-nicotinamide.
   6-fluoro-N-(4-(1-methylethyl)phenyl)-2-((4-pyridinylmethyl)amino)-3-pyridinecarboxamide;
   2-((4-pyridinylmethyl)amino)-N-(3-(((2S)-2-pyrrolidinylmethyl)oxy)-5-(trifluoromethyl)
   phenyl)-3-pyridinecarboxamide;
   N-(3-(1,1-dimethylethyl)-1H-pyrazol-5-yl)-2-((4-pyridinylmethyl)amino)-3-pyridinecarboxamide;
   N-(3,3-dimethyl-2,3-dihydro-1-benzofuran-6-yl)-2-((4-pyridinylmethyl)amino)-3-pyridinecarboxamide;
   N-(3-((((1S)-1-methyl-2-pyrrolidinyl)methyl)oxy)-5-(triffuoromethyl)phenyl)-2-((4-pyridinylmethyl)amino)-3-pyridineoarboxamide;
   2-((4-pyridinymethyl)apmino)-N-(3-((2-(1-pyrrolidinyl)ethyl)oxy)-4-(trifluoromothyl)phenyl)-3-pyridinecarboxamide;
   N-(3,3-dimethyl-2,3-dihydro-1H-indol-6-yl)-2-((4-pyridinylmethyl)amino)-3-pyridinecarboxamide;
   N-(4-(pentafluoroethyl)-3-(((2S)-2-pyrrolidinylmethyl)oxy)phenyl)-2((4-pyridinylmethyl)amino)-3-pyridinecarboxamide;
   N-(3-((3-azetidinylmethyl)oxy)-5-(trifluoromethyl)phanyl)-2-((4-pyridinylmethyl) amino)-3-pyridinecarboxamide;
   N-(3-(4-piperidinyloxy)-5-(trifluotomethyl)phenyl)-2-((2-(3-pyridinyl)ethyl)amino)-3-pyridinecarboxamide;
   N-(4,4-dimethyl-1,2,3,4-tetrahydro-isoquinolin-7-yl)-2-(1H-indazol-6-ylamino)-nicotinamide;
   2-(1H-indazol-6-ylamino)-N-[3-(1-methylpyrrolidin-2-ylmethoxy)-5-tifluoromethyl-phenyl]-nicotinamide;
   N-[1-2-dimethylamino-acetyl)-3,3-dimethyl-2,3-dihydro-1H-indol-6-yl]-2-(1H-indazol-6-ylamino)-nicolinamide;
   2-(1H-indazol-6-ylamino)-N-[3-(pyrrolidin-2-ylmethoxy)-5-trifluoromethyl-phenyl]-nicotinamide:
   N-(1-acetyl-3,3-dimethyl-2,3-dihydro-1H-indol-6-yl)-2-(1H-indazol-6-ylamino)-nicotinamide;
   N-(4,4-dimethyl-1-oxo-1,2,3,4-tetrahydro-isoquinolin-7-yl)-2-(1H-indazol-6-ylamino)-nicotinamide;
   N-[4-(tert-butyl)-3-(3-piperidylpropyl)phenyl][2-(1H-indazol-6-ylamino)(3-pyridyl)] carboxamide;
   N-[5-(tert-butyl)isoxazol-3-yl][2-(1H-indazol-6-ylamino)(3-pyridyl)]carboxamide; and
   N-[4-(tert-butyl)phenyl][2-(1H-indazol-6-ylamino)(3-pyridyl)]carboxamide.

The invention describes co-therapy with the VEGFR inhibitor AMG706.

The invention describes co-therapy with VEGFR inhibitors including Nexavar (Bayer BAY 43-9006), Astra Zeneca AZ 2171, Novartis/Schering PTK/ZK, PTK787/ZK 222584, Pfizer AG-13736 and Sutent (Pfizer SU11248).

Other VEGFR inhibitors described in the following patents and patent applications can be used in combination therapy: US 6,258,812, US 2003/0103091. WO 01/37820, US 6,235,764, WO 01/32651, US 6,630,500, US 6,515,004, US 6,713,485, US 5,521,184, US 5,770,599, US 5,747,498, WO 02168406, WO 02166470, WO 02155501, WO 04105279, WO 04/07481, WO 04/07458, WO 04/09784, WO 02/59110, WO 99/45009, WO 00/59509, WO 99/61422, US 5,990,141, WO 00/12089 and WO 00/02871,

The invention describes co-therapy with VEGFR inhibitors described in US 2003/0125339

The invention also describes co-therapy with VEGFR inhibitors described in US 2003/0125339 or US 2003/0225106

The invention also describes co-therapy with VEGFR inhibitors described in WO 00/42012, WO 00/41698, US 2005/0038080A1, US 2003/0125359A1, US 2002/0165394A1. US 2001/003447A1, US 2001/0016659A1, and US 2002/013774A1 particularly in parts disclosing the foregoing VBGF inhibitors.

The invention also describes humanized or fully human EGFR antibodies.

The invention also describes EGFR inhibitory agents (e.g., antibodies or antigen binding regions that specifically bind thereto) such as panitumumab, ERBITU^{™} (Cetuximab), EMD72000, TheraCIM bR3 or LICR 806.

Other EGFR antibodies described in 6,235,883 can be used in combination therapy.

The invention also describes co-therapy with panitumumab.

The invention also describes a kit comprising, in one or more containers, separately or in admixture one or more EGFR antibodies inhibitors and one or more VEGF inhibitors in accordance with any of the foregoing.

The invention also describes kit, wherein the inhibitors are comprised in pharmaceutically acceptable formulations.

The invention also describes a kit, comprising panitumumab and AMG 706.

The invention also describes a kit, wherein the inhibitors are disposed in separate containers.

The invention also describes a kit according to any of the foregoing, further comprising integrally thereto or as one or more separate documents, information pertaining to the contents or the kit and the use of the inhibitors.

The invention also describes a kit according to any of the foregoing, wherein the compositions are formulated for reconstitution in a diluent,

The invention also describes a kit according to any of the foregoing, further comprising a container of sterile diluent.

The invention also describes a kit according to any of the foregoing, wherein said compositions are disposed in vials under partial vacuum sealed by a septum and suitable for reconstitution to form a formulation effective for parental administration.

As used in relation to the invention, the term "treating" or "treatment" and the like should be taken broadly. They should not be taken to imply that an animal is treated to total recovery. Accordingly, these terms include amelioration of the symptoms or severity of a particular condition or preventing or otherwise reducing the risk of further development of a particular condition.

The term "comprising" is meant to be open ended, including the indicated component but not excluding other elements.

The phrase "therapeutically-effective" is intended to qualify the amount of each agent, which will achieve the goal of improvement in disorder severity and the frequency of incidence over treatment of each agent by itself, while avoiding adverse side effects typically associated with alternative therapies. For example, effective neoplastic therapeutic agents prolong the survivability of the patient, inhibit the rapidly-proliferating cell growth associated with the neoplasm, or effect a regression of the neoplasm.

It should be appreciated that uses of the invention may be applicable to various species of subjects, preferably mammals, more preferably humans.

As used herein, the compounds of the present invention include the pharmaceutically acceptable derivatives thereof.

Where the plural form is used for compounds, salts, and the like, this is taken to mean also a single compound, salt and the like.

A "pharmaceutically-acceptable derivative" denotes any salt, ester of a compound of this invention, or any other compound which upon administration to a patient is capable of providing (directly or indirectly) a compound of this invention, or a metabolite or residue thereof.

The terms "cancer" and "cancerous" when used herein refer to or describe the physiological condition in mammals that is typically characterized by unregulated cell growth. Examples of cancer include but are not limited to, carcinoma, lymphoma, sarcoma, blastoma and leukemia More particular examples of such cancers include squamous cell carcinoma, lung cancer, pancreatic cancer, cervical cancer, bladder cancer, hepatoma, breast cancer, colon carcinoma, and head and neck cancer.

A VEGFR inhibitor is defined as a compound with a molecular weight less than about 1000 that inhibits the receptor as shown with in vitro testing or by other means.

The following are among specific VEGF inhibitors that are described in this regard:
AEE-788 (Novartis) (also called AE-788 and NVP-AEE-788, among others) including formulations for oral administration and closely related VEGF inhibitors;
AG-13736 (Pfizer) (also called AG-013736) including formulations for oral administration and closely related VEGF inhibitors;
AC-028262 (Pfizer) and closely related VEGF inhibitors;
AVE-8062 (Ajinomoto Co. and Sanofi-aventis) (also called AC-7700 and combretastatin A4 analog, among others), and closely related VEGF inhibitors;
AZD.2171 (AstraZeneca) and closely related VEGF inhibitors;
Nexavar® (Bayer AG and Onyx) (also called CAS Registry Number 284461-73-0, BAY-43-9006, raf kinase inhibitor, sorafenib, sorafenib analogs, and IDDBCP150446, among others) and closely related VEGP inhibitors;
BMS-387032 (Sunesis and Bristol-Myers Squibb) (also called SNS-032 and CAS Registry Number 345627-80-7, among others) and closely related VEGF inhibitors;
CEP-7055 (Cephalon and Sanofi-aventis) (also called CEP-11981 and SSR-106462, among others) and closely related VEGF inhibitors;
CHIR-258 (Chiron) (also called CAS Registry Number 405169-16-6, GFKI, and GFKI-258, among others) and closely related VEGF inhibitors;
CP-547632 (OSI Pharmaceuticals and Pfizer) (also called CAS Registry Number 252003-65-9, among others) and closely related VEGF inhibitors such as, for instance, CP-564959;
B-7080 (Eisai Co.) (also called CAS Registry Number 417716-92-8 and ER-203492-00, among others) and closely related VBGF inhibitors;
786034 (GlaxoSmithKline) and closely related VEGF inhibitors;
GW-654652 (GlaxoSmithKline) and closely related indazolylpyrimidine Kdr inhibitors;
KRN-951 (Kirin Brewery Co.) and other closely related quinoline-urea VEGF inhibitors;
PKC-412 (Novartis) (also called CAS Registry Number 120685-11-2, benzoylstaurosporine, CGP-41251, midostaurin, and SIT-412, among others) and closely related VEGF inhibitors;
PTK-787 (Novartis and Schering) (also called CAS Registry Numbers 212141-54-3 and 212142-18-2, PTK/ZK, PTK-787/ZK-222584, ZK-22584, VEGF-TKI. VEGF-RKI, PTK-787A, DE-00268, CGP-79787, CGP-79787D, vatalanib, ZK-222584, among others) and closely related anilinophthalezine derivative VEGF inhibitors;
SU11248 (Sugen and Pfizer) (also called SU-11248, SU-011248, SU-11248J, Sutent®, and sunitinib malate, among others) and closely related VEGF inhibitors;
SU-5416 (Sugen and Pfizer/Pharmacia) (also called CAS Registry Number 194413-58-6, semaxanib, 204005-46-9, among others) and closely related VEGF inhibitors;
SU-6668 (Sugen and Taiho) (also called CAS Registry Number 252916-29-3, SU-006668, and TSU-68, among others) and closely related VEGF inhibitors as described in, among others, WO 99/48868, WO 99161422, and WO 00/38519, particularly in parts pertaining to SU-6668 and closely related VEGF inhibitors, their structures and properties, and methods for making and using them;
Thalidomide (Celgone) (also called CAS Registry Number 50-35-1, Synovir, Thalidomide Pharmion, and Thalomid, among others) and closely related VEGF inhibitors;
XL-647 (Exelixis) (also called EXEL-7647, among others) and closely related VEGF inhibitors;
XL-999 (Exelixis) (also called EL-0999, among others) and closely related VEGF inhibitors;
XL-880 (Exelixis) (also called EXEL-2880, among others) and closely related VEGF inhibitors;
ZD-6474 (AstraZeneca) (also called CAS Registry Number 443913-73-3, Zactima, and AZD-6474, among others) and closely related anilinoquinazoline VEGF inhibitors; and
ZK-304709 (Schering) (also called CDK inhibitors (indirubin derivatives), ZK. CDK, MTGI, and multi-target tumor growth inhibitor, among others) and other closely related compounds including the indirubin derivative VEGF inhibitors described in WO 001234717, WO 021074742, WO 02/100401, WO 00/244148, WO 02/096888, WO 03/029223, WO 02/092079, and WO 02/094814 particularly in parts pertinent to these and closely related VEGF inhibitors, their structures and properties, and methods for making and using them.

Also among VEGF inhibitors in this regard are: Pazopanib, CDP791, Enzastaurin, BIBF 1120, BAY 573952, BAY 734506, XL 184, IMC-1121B, CEP 701, SU 014813, SU 10944, SU 12662, OSI-930, and BMS 582664, and closely related VEGF inhibitors.

In addition to the foregoing inhibitors that act directly on VBGF or VEGFR, the following inhibitors have anti-angiogenic properties and can be used in much the same way as inhibitors that act directly:
ZD-6126 (AstraZeneca and Angiogene) (also called CAS Registry Number 219923-05-4, N-acetylcolchinol phosphate, ANG-453, AZD-6126, ZD-6126 derivatives and ZM-445526, among others) and closely related VEGF inhibitors such as other inhibitors in the ANG-400 series;
Imatinib (Novartis) (also called CAS Registry Numbers 152459-95-5 and 220127-57-1, Glivec, Gleeveo, STI-571, and CGP-57148, among others) and closely related VEGF inhibitors;
RAD-001 (Novartis) (also called CAS Registry Number 159351-69-6, RAD-001, SDZ-RAD, Certican, and everolimus, among others) and closely related VEGF inhibitors; and
BMS-354825 (Bristol-Myers Squibb) (also called CAS Registry Number 302962-49-8, Src/Abl kinase inhibitor, and dasatinib, among others) and closely related VEGF inhibitors.

Also useful in this are regard are CCI-779,17-AAG, DMXAA, CI-1040, and CI-1033.

Among the described VEGF inhibitors preferred are the following: (a) a compound described in US 2003/0125339 particularly in parts disclosing VEGF inhibitors; (b) a substituted alkylamine derivative described inUS 2003/0125339 or US 2003/0225106 particularly in parts disclosing VEGF inhibitors; (c) a substituted omega-carboxyaryl diphenyl urea or derivative thereof as described in WO 00/42012, WO 00/41698, US 2005/0038080A1, US 2003/0125359A1, US 2002/0165394A1, US 2001/003447A1, US 2001/0016659A1. and US 2002/013774A1 particularly in parts disclosing the foregoing VEGF inhibitors; (d) an anilinophthalazine or derivative thereof that binds to and inhibits the activity of multiple receptor tyrosine kinases including binding to the protein kinase domain and inhibition of VEGFR1 and VEGFR2; and (e) (5-[5-fluoro-2-oxo-1,2-dihydroindol-(3Z)-ylidenemethyl]-2,4-dimethyl-1*H-*pyrrole-3-carboxylic acid [2-dicthylaminoethyl]amide) or derivative thereof that are VEGF inhibitors.

In this regard, certain of the very highly particularly preferred VEGF inhibitors are further described below,
(1) AMG 706
(2) Nexavar
(3) AZD-2171
(4) AC-13736
(5) PTK/ZK and
(6) Sutent.

Among these AMG 706 is among the most highly preferred VEGF inhibitors.

"Nexevar®" (also known as BAY 43-9006, sorafenib, CAS Registry Number 284461-73-0, raf kinase inhibitor, sorafenib analogs, and IDDBCP150446, among others) is a substituted omega carboxy diphenyl urea that inhibits RAF-1 activation, and thereby decreases Ray-1 dependent phosphorylation of MEK-1 and ERK-1, as described in US Patent Application No. 2003/0125359A1, WO03/047523A2, and Wilhelm et al., Current Pharmaceutical Design, vol. 8, pp. 2255-2257 (2002), particularly in parts pertinent to Nexavar®, its structure and properties, methods for making and using it, and other related molecules. Its chemical name is 4-(4-{3-[4-Chloro-3-(trifluoromethyl)phenyl]ureido} phenoxy)-N²-methylpyridine-2-carboxamide. A variety of derivatives have been produced. Among these are fluorinated derivatives described in US 2005/0038080A1 and WO 2005/009961A2, particularly as to these and other pharmaceutically active diphenyl urea compounds

"PTK/ZK," also known as vatalanib, is a multi-YEGF receptor tyrosine kinase inhibitor that is said to block tumor angiogenesis and lymphangiogenesis. Its chemical name is N-(4-chlorophenyl)-4-(pyridin-4-ylmethyl)phthalazin-1-amine. It also is known as CAS Registry Numbers 212141-54-3 and 212142.18-2. PTK787, PTX787/ZK, PTK-787/ZK-222584, PTK787/2K222584, ZK-22584, VEGF-TKI, VEGP-RKI, PTK-787A, DE-00268, CGP-79787, CGP-79787D, vatalanib, and ZK-222584. See Thomas, A., et al., J. of Clin. Oncology, 23(18):4162-4171 (2005); US 2005/0118600A1, particularly as to the structure, synthesis, properties, and uses of PTK/ZK and related compounds.

"Sutent®" is a smell molecule receptor tyrosine kinase inhibitor with the chemical name (5-[5-fluoro-2-oxo-1,2-dihydroindol-(3Z)-ylidenemethyl]-2,4-dimethyl-1*H-*pyrrole-3-carboxylic acid [2-diethylaminoethyl]amide). Sutent® is also known as sunitinib malate, SU11248, SU-11248, SU-011248, and SU-11248J, and is reported to have anti-angiogenic and anti-tumor activities. See Mendel, D., et al., Clinical Cancer Research, 9:327-337 (2003); Schlessingar, J., The Scientist, 19(7):17 (2005), particularly as to the structure, synthesis, properties, and uses of Sutent® and related compounds.

"AMG 706" is a multi-kinase inhibitor that interferes with the Kit, Ret, FDGF, and VEGF-signalling pathways, as described in U.S. Patent No. 6,995,162, particularly in parts pertinent to AMG 706, its structure and properties, methods for making and using it, and other related compounds. Its chemical name is N-(2,3-dihydro-3,3-dimethyl-1H-indol-6-yl)-2-[(4-pyridinylmethyl)amino]-3-pyridinecarboxamide, AMG 706 is also occasionally referred to as VEGF inhibitor B in this application. As used herein the term AMG 706 includes pharmaceutically acceptable salts, in particular, the diphosphate salt, except as otherwise provided herein.

An EGFR antibody is defined as an Antibody, or fragment thereof, that interferes with the binding between EGF and EGFR, as shown with in vitro testing or by other means. Cetuximab is also occasionally referred to as EGF inhibitor B in this application.

"Panitumumab" is a EGFR antibody, as described in U.S. Patent No. 6,235,883, WO 03/99205 and US 2005/0241006 Panitumumab is also occasionally referred to as EGF inhibitor A in this application.

A "pharmaceutically-acceptable derivative" denotes any salt, ester of a compound of this invention, or any other compound which upon administration to a patient is capable of providing (directly or indirectly) a compound of this invention, or a metabolite or residue thereof.

The term "pharmaceutically-acceptable salts" embraces salts commonly used to form alkali metal salts and to form addition salts of free acids or free bases. The nature of the salt is not critical, provided that it is pharmaceutically-acceptable. Suitable pharmaceutically-acceptable acid addition salts may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, arylaliphatic, heterocyclic, carboxylic and sulfonic classes of organic acids, example of which are formic, acetic, adipic, butyric, propionic, succinic, glycolic, gluconic, lactic, malic, tartaric, citric, ascorbic, glucuronic, maleic, fumaric, pyruvic, aspartic, glutamic, benzoic, anthranilic, mesylic, 4-hydroxybenzoic, phenylacetic, mandelic, embonic (pamoic), methanesulfonic, ethanesulfonic, ethanedisulfonic, benzenesulfonic, pantothenic, 2-hydroxyethanesulfonic, toluenesulfonic, sulfanilic, cyclohexylaminosulfonic, camphoric, camphorsulfonic, digluconic, cyclopentanepropionic, dodecylsulfonic, glucoheptanoic, glycerophosphonic, heptanoic, hexanoic, 2-hydroxy-ethanesulfonic, nicotinic, 2-naphthalenesulfonic, oxalic, palmoic, pectinic, persulfuric, 2-phenylpropionic, picric, pivalic propionic, succinic, tartaric, thiocyanic, mesylic, undecanoic, stearic, algenic, β-hydroxybutyric, salicylic, galactaric and galacturonic acid. Suitable pharmaceutically-acceptable base addition salts include metallic salts, such as salts made from aluminum, calcium, lithium, magnesium, potassium, sodium and zinc, or salts made from organic bases including primary, secondary and tertiary amines, substituted amines including cyclic amines, such as caffeine, arginine, diethylamine, N-ethyl piperidine, aistidine, glucamine, isopropylamine, lysine, morpholine, N-ethyl morpholine, piperazine, piperidine, triethylamine, trimethylamine. All of these salts may be prepared by conventional means from the corresponding compound of the invention by reacting, for example, the appropriate acid or base with the compound of the invention. When a basic group and an acid group are present in the same molecule, a compound of the invention may also form internal salts.

Currently, standard treatment of primary tumors consists of surgical excision followed by either radiation or IV administered chemotherapy. The typical chemotherapy regime consists of either DNA alkylating agents, DNA intercalating agents, CDK inhibitors, or microtubule poisons. The chemotherapy doses used are just below the maximal tolerated dose and therefore dose limiting toxicities typically include, nausea, vomiting, diarrhea, hair loss, neutropenia and the like.

There are large numbers of antineoplastic agents available in commercial use, in clinical evaluation and in pre-clinical development, which would be selected for treatment of neoplasia by combination drug chemotherapy. Such antineoplastic agents fall into several major categories, namely, antibiotic-type agents, alkylating agents, antimetabolite agents, hormonal agents, immunological agents, interferon-type agents and a category of miscellaneous agents.

A first family of antineoplastic agents which may be used in combination with compounds of the present invention consists of antimetabolite-type/thymidilate synthase inhibitor antineoplastic agents. Suitable antimetabolite antineoplastic agents may be selected from but not limited to the group consisting of 5-FU, fibrinogen, acanthifolic acid, aminothiadiazole, brequinar sodium, carmofur, Ciba-Geigy CGP-30694, cyclopentyl cytosine, cytarabine phosphate stearate, cytarabine conjugates, Lilly DATHF, Merrel Dow DDFC, dezaguanine, dideoxycytidine, dideoxyguanosine, didox, Yoshitomi DMDC, doxifluridine, Wellcome EHNA, Merck & Co. EX-015, fazarabine, floxuridine, fludarabine phosphate, 5-fluorouracil, N-(2'-furanidyl)-5-fluorouracil, Daiichi Seiyaku FO-152, isopropyl pyrrolizine, Lilly LY-188011, Lilly LY-264618, methobenzaprim, methotrexate, Wellcome MZPES, norspermidine, NCI NSC-127716, NCI NSC-264880, NCI NSC-39661, NCI NSC-612567, Warner-Lambert PALA, pentostatin, piritrexim, plicamycin, Asahi Chemical PL-AC, Takeda TAC-788, thioguanine, tiazofurin, Erbamont TIF, trimetrexate, tyrosine kinase inhibitors, Taiho UFT and uricytin.

A second family of antineoplastic agents which may be used in combination with compounds of the present invention consists of alkylating-type antineoplastic agents. Suitable alkylating-type antineoplastic agents may be selected from but not limited to the group consisting of Shionogi 254-S, aldo-phosphamide analogues, altretamine, anaxirone, Boehringer Mannheim BBR-2207, bestrabucil, budotitane, Wakunaga CA-102, carboplatin, carmustine, Chinoin-139, Chinoin-153, chlorambucil, cisplatin, cyclophosphamide, American Cyanamid CL-286558, Sanofi CY-233, cyplatate, Degussa D-19-384, Sumimoto DACHP(Myr)2, diphenylspiromustine, diplatinum cytostatic, Erba distamycin derivatives, Chugai DWA-2114R, ITI E09, elmustine, Erbamont FCE-24517, estramustine phosphate sodium, fotemustine, Unimed G-6-M, Chinoin GYKI-17230, hepsul-fam, ifosfamide, iproplatin, lomustine, mafosfamide, mitolactol, Nippon Kayaku NK-121, NCI NSC-264395, NCI NSC-342215, oxaliplatin, Upjohn PCNU, prednimustine, Proter PTT-119, ranimustine, semustine, SmithKline SK&F-101772, Yakult Honsha SN-22, spiromus-tine, Tanabe Seiyaku TA-077, tauromustine, temozolomide, teroxirone, tetraplatin and trimelamol.

A third family of antineoplastic agents which may be used in combination with compounds of the present invention consists of antibiotic-type antineoplastic agents. Suitable antibiotic-type antineoplastic agents may be selected from but not limited to the group consisting ofTaiho 4181-A, aclarubicin, actinomycin D, actinoplanone, Erbamont ADR-456, aeroplysinin derivative, Ajinomoto AN-201-II, Ajinomoto AN-3, Nippon Soda anisomycins, anthracycline, azino-mycin-A, bisucaberin, Bristol-Myers BL-6859, Bristol-Myers BMY-25067, Bristol-Myers BMY-25551, Bristol-Myers BMY-26605, Bristol-Myers BMY-27557, Bristol-Myers BMY-28438, bleomycin sulfate, bryostatin-1, Taiho C-1027, calichemycin, chromoximycin, dactinomycin, daunorubicin, Kyowa Hakko DC-102, Kyowa Hakko DC-79, Kyowa Hakko DC-88A, Kyowa Hakko DC89-A1, Kyowa Hakko DC92-B, ditrisarubicin B, Shionogi DOB-41, doxorubicin, doxorubicin-fibrinogen, elsamicin-A, epirubicin, erbstatin, esorubicin, esperamicin-A1, esperamicin-Alb, Erbamont FCE-21954, Fujisawa FK-973, fostriecin, Fujisawa FR-900482, glidobactin, gregatin-A, grincamycin, herbimycin, idarubicin, illudins, kazusamycin, kesarirhodins, Kyowa Hakko KM-5539, Kirin Brewery KRN-8602, Kyowa Hakko KT-5432, Kyowa Hakko KT-5594, Kyowa Hakko KT-6149, American Cyanamid LL-D49194, Meiji Seika ME 2303, menogaril, mitomycin, mitoxantrone, SmithKline M-TAG, neoenactin, Nippon Kayaku NK-313, Nippon Kayaku NKT-01, SRI International NSC-357704, oxalysine, oxaunomycin, peplomycin, pilatin, pirarubicin, porothramycin, pyrindanycin A, Tobishi RA-I, rapamycin, rhizoxin, rodorubicin, sibanomicin, siwenmycin, Sumitomo SM-5887, Snow Brand SN-706, Snow Brand SN-07, sorangicin-A, sparsomycin, SS Pharmaceutical SS-21020, SS Pharmaceutical SS-7313B, SS Pharmaceutical SS-9816B, steffimycin B, Taiho 4181-2, talisomycin, Takeda TAN-868A, terpentecin, thrazine, tricrozarin A, Upjohn U-73973, Kyowa Hakko UCN-10028A, Fujisawa WF-3405, Yoshitomi Y-25024 and zorubicin.

A fourth family of antineoplastic agents which may be used in combination with compounds of the present invention consists of a miscellaneous family of antineoplastic agents, including tubulin interacting agents, topoisomerase **II** inhibitors, topoisomerase I inhibitors and hormonal agents, selected from but not limited to the group consisting of α-carotene, α-difluoromethyl-arginine, acitretin, Biotec AD-5, Kyorin AHC-52, alstonine, amonafide, amphethinile, amsacrine, Angiostat, ankinomycin, anti-neoplaston A10, antineoplaston A2, antineoplaston A3, antineoplaston A5, antineoplaston AS2-1, Henkel APD, aphidicolin glycinate, asparaginase, Avarol, baccharin, batracylin, benfluron, benzotript, Ipsen-Beaufour BIM-23015, bisantrene, Bristol-Myers BMY-40481, Vestar boron-10, bromofosfamide, Wellcome BW-502, Wellcome BW-773, caracemide, carmethizole hydrochloride, Ajinomoto CDAF, chlorsulfaquinoxalone, Chemes CHX-2053, Chemex CH2-100, Warner-Lambert CI-921, Warner-Lambert CI-937, Warner-Lambert CI-941, Warner-Lambert CI-958, clanfenur, claviridenone, ICN compound 1259, ICN compound 4711, Contracan, Yakult Honsha CPT-11, crisnatol, curaderm, cytochalasin B, cytarabine, cytocytin, Merz D-609, DABIS maleate, dacarbazine, datelliptinium, didemnin-B, dihaematoporphyrin ether, dihydrolenperone, dinaline, distamycin, Toyo Pharmar DM-341, Toyo Pharmar DM-75, Daiichi Seiyaku DN-9693, docetaxel elliprabin, elliptinium acetate, Tsumura EPMTC, the epothilones, ergotamine, etoposide, etretinate, fenretinide, Fujisawa FR-57704, gallium nitrate, genkwadaphnin, Chugai GLA-43, Glaxo GR-63178, grifolan NMF-5N, hexadecylphosphocholine, Green Cross HO-221, homoharringtonine, hydroxyurea, BTG ICRF-187, ilmofosine, isoglutamine, isotretinoin, Otsuka JI-36, Ramot K-477, Otsuak K-76COONa, Kureha Chemical K-AM, MECT Corp KI-8110, American Cyanamid L-623, leukoregulin, lonidamine, Lundbeck LU-23-112, Lilly LY-186641, NCI (US) MAP, marycin, Merrel Dow MDL-27048, Medco MEDR-340, merbarone, merocyanine derivatives, methylanilinoacridine, Molecular Genetics MGI-136, minactivin, mitonafide, mitoquidone mopidamol, motretinide, Zenyaku Kogyo MST-16, N-(retinoyl)amino acids, Nisshin Flour Milling N-021, N-acylated-dehydroalanines, nafazatrom, Taisho NCU-190, nocodazole derivative, Normosang, NCI NSC-145813, NCI NSC-361456, NCI NSC-604782, NCI NSC-95580, ocreotide, Ono ONO-112, oquizanocine, Akzo Org-10172, paclitaxel, pancratistatin, pazelliptine, Warner-Lambert PD-111707, Warner-Lambert PD-115934, Warner-Lambert PD-131141, Pierre Fabre PE-1001, ICRT-peptide D, piroxantrone, polyhaematoporphyrin, polypreic acid, Efamol porphyrin, probimane, procarbazine, proglumide, Invitron protease nexin I, Tobishi RA-700, razoxane, Sapporo -Breweries RBS, restriction-P, retelliptine, retinoic acid, Rhone-Poulenc RP-49532, Rhone-Poulenc RP-56976, SmithKline SK&F-104864, Sumitomo SM-108, Kuraray SMANCS, SeaPharm SP-10094, spatol, spirocyclopropane derivatives, spirogermanium, Unimed, SS Pharmaceutical SS-554, strypoldinone, Stypoldione, Suntory SUN 0237, Suntory SUN 2071, superoxide dismutase, Toyama T-506, Toyama T-680, taxol, Teijin TEI-0303, teniposide, thaliblastine, Eastman Kodak TJB-29, tocotrienol, topotecan, Topostin, Teijin TT-82, Kyowa Hakko UCN-01, Kyowa Hakko UCN-1028, ukrain, Eastman Kodak USB-006, vinblastine sulfate, vincristine, vindesine, vinestramide, vinorelbine, vintriptol, vinzolidine, withanolides and Yamanouchi YM-534.

Alternatively, the present compounds may also be used in co-therapies with other anti-neoplastic agents, such as acemannan, aclarubicin, aldesleukin, alemtuzumab, alitretinoin, altretamine, amifostine, aminolevulinic acid, amrubicin, amsacrine, anagrelide, anastrozole, ANCER, ancestim, ARGLABIN, arsenic trioxide, BAM 002 (Novelos), bexarotene, bicalutamide, broxuridine, capecitabine, celmoleukin, cetrorelix, cladribine, clotrimazole, cytarabine ocfosfate, DA 3030 (Dong-A), daclizumab, denileukin diftitox, deslorelin, dexrazoxane, dilazep, docetaxel, docosanol, doxercalciferol, doxifluridine, doxorubicin, bromocriptine, carmustine, cytarabine, fluorouracil, HIT diclofenac, interferon alfa, daunorubicin, doxorubicin, tretinoin, edelfosine, edrecolomab, eflornithine, emitefur, epirubicin, epoetin beta, etoposide phosphate, exemestane, exisulind, fadrozole, filgrastim, finasteride, fludarabine phosphate, formestane, fotemustine, gallium nitrate, gemcitabine, gemtuzumab zogamicin, gimeracil/oteracil/tegafur combination, glycopine, goserelin, heptaplatin, human chorionic gonadotropin, human fetal alpha fetoprotein, ibandronic acid, idarubicin, (imiquimod, interferon alfa, interferon alfa, natural, interferon alfa-2, interferon alfa-2a, interferon alfa-2b, interferon alfa-N1, interferon alfa-n3, interferon alfacon-1, interferon alpha, natural, interferon beta, interferon beta-1a, interferon beta-1b, interferon gamma, natural interferon gamma-1a, interferon gamma-1b, interleukin-1 beta, iobenguane, irinotecan, irsogladine, lanreotide, LC 9018 (Yakult), leflunomide, lenograstim, lentinan sulfate, letrozole, leukocyte alpha interferon, leuprorelin, levamisole + fluorouracil, liarozole, lobaplatin, lonidamine, lovastatin, masoprocol, melarsoprol, metoclopramide, mifepristone, miltefosine, mirimostim, mismatched double stranded RNA, mitoguazone, mitolactol, mitoxantrone, molgramostim, nafarelin, naloxone + pentazocine, nartograstim, nedaplatin, nilutamide, noscapine, novel erythropoiesis stimulating protein, NSC 631570 octreotide, oprelvekin, osaterone, oxaliplatin, paclitaxel, pamidronic acid, pegaspargase, peginterferon alfa-2b, pentosan polysulfate sodium, pentostatin, picibanil, pirarubicin, rabbit antithymocyte polyclonal antibody, polyethylene glycol interferon alfa-2a, porfimer sodium, raloxifene, raltitrexed, rasburicase, rhenium Re 186 etidronate, RII retinamide, rituximab, romurtide, samarium (153 Sm) lexidronam, sargramostim, sizofiran, sobuzoxane, sonermin, strontium-89 chloride, suramin, tasonermin, tazarotene, tegafur, temoporfin, temozolomide, teniposide, tetrachlorodecaoxide, thalidomide, thymalfasin, thyrotropin alfa, topotecan, toremifene, tositumomab-iodine 131, trastuzumab, treosulfan, tretinoin, trilostane, trimetrexate, triptorelin, tumor necrosis factor alpha, natural, ubenimex, bladder cancer vaccine, Maruyama vaccine, melanoma lysate vaccine, valrubicin, verteporfin, vinorelbine, VIRULIZIN, zinostatin stimalamer, or zoledronic acid; abarelix; AE 941 (Aeterna), ambamustine, antisense oligonucleotide, bcl-2 (Genta), APC 8015 (Dendreon), cetuximab, decitabine, dexaminoglutethimide, diaziquone, EL 532 (Elan), EM 800 (Endorecherche), eniluracil, etanidazole, fenretinide, filgrastim SD01 (Amgen), fulvestrant, galocitabine, gastrin 17 immunogen, HLA-B7 gene therapy (Vical), granulocyte macrophage colony stimulating factor, histamine dihydrochloride, ibritumomab tiuxetan, ilomastat, IM 862 (Cytran), interleukin-2, iproxifene, LDI 200 (Milkhaus), leridistim, lintuzumab, CA 125 MAb (Biomira), cancer MAb (Japan Pharmaceutical Development), HER-2 and Fc MAb (Medarex), idiotypic 105AD7 MAb (CRC Technology), idiotypic CEA MAb (Trilex), LYM-1-iodine 131 MAb (Techniclone), polymorphic epithelial mucin-yttrium 90 MAb (Antisoma), marimastat, menogaril, mitumomab, motexafin gadolinium, MX 6 (Galderma), nelarabine, nolatrexed, P 30 protein, pegvisomant, pemetrexed, porfiromycin, prinomastat, RL 0903 (Shire), rubitecan, satraplatin, sodium phenylacetate, sparfosic acid, SRL 172 (SR Pharma), SU 5416 (SUGEN), TA 077 (Tanabe), tetrathiomolybdate, thaliblastine, thrombopoietin, tin ethyl etiopurpurin, tirapazamine, cancer vaccine (Biomira), melanoma vaccine (New York University), melanoma vaccine (Sloan Kettering Institute), melanoma oncolysate vaccine (New York Medical College), viral melanoma cell lysates vaccine (Royal Newcastle Hospital), or valspodar.

Alternatively, the present compounds may also be used with radiation. Alternatively, the present compounds may also be used in conjunction with agents used for hormonal therapy, such as for treatment of breast and prostate cancer. Examples include aromatase inhibitors (e.g. Arimidex (chemical name: anastrozole), Aromasin (chemical name: exemestane), and Femara (chemical name: letrozole)); Serms (selective estrogen-receptor modulators) such as tamoxifen; and ERDs (estrogen-receptor downregulators), e.g. Faslodex (chemical name: fulvestrant).

As will be appreciated, the dose of an combination of the present invention to be administered, the period of administration, and the general administration regime may differ between subjects depending on such variables as the severity of symptoms, the type of tumor to be treated, the mode of administration chosen, type of composition, size of a unit dosage, kind of excipients, the age and/or general health of a subject, and other factors well known to those of ordinary skill in the art.

Administration may include a single daily dose or administration of a number of discrete divided doses as may be appropriate. An admimstration regime may also include administration of one or more of the active agents, or compositions comprising same, as described herein. The period of administration may be variable.

It may occur for as long a period is desired.

Administration may include simultaneous administration of suitable agents or compositions or sequential administration of agents or compositions.

### FORMULATIONS

Also described is a class of pharmaceutical compositions comprising the active VEGFR inhibitors in association with one or more non-toxic, pharmaceutically-acceptable carriers and/or diluents and/or adjuvants (collectively referred to herein as "carrier" materials) and, if desired, other active ingredients. The active compounds of the medicament may be administered by any suitable route, preferably in the form of a pharmaceutical composition adapted to such a route, and in a dose effective for the treatment intended. The compositions of the present invention may, for example, be administered orally, mucosally, topically, rectally, pulmonarily such as by inhalation spray, or parentally including intravascularly, intravenously, intraperitoneally, subcutaneously, intramuscularly intrasternally and infusion techniques, in dosage unit formulations containing conventional pharmaceutically acceptable carriers, adjuvants, and vehicles.

The medicaments of this invention can be processed in accordance with conventional methods of pharmacy to produce medicinal agents for administration to patients, including humans and other mammals.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. For example, these may contain an amount of active ingredient from about 1 to 2000 mg, preferably from about 1 to 500 mg. A suitable daily dose for a human or other mammal may vary widely depending on the condition of the patient and other factors, but, once again, can be determined using routine methods. For example dosages from about 10 mg to about 150 mg, or about 25 to about 125 mg may be used. The therapeutically effective amount of VEGFR inhibitor in the composition can be chosen to be about 25 mg, about 50 mg, about 75 mg, about 100 mg, about 125 mg, or about 150 mg. The therapeutically effective amount of VEGFR inhibitor in the composition can be chosen to be about 50 mg dosed twice a day, or about 75 mg dosed twice a day, or about 100 mg dosed twice a day, or about 100 mg dosed once a day, or about 125 mg dosed once a day.

The amount of compounds which are administered and the dosage regimen for treating a disease condition with the compositions of this invention depends on a variety of factors, including the age, weight, sex and medical condition of the subject, the type of disease, the severity of the disease, the route and frequency of administration, and the particular compound employed. Thus, the dosage regimen may vary widely, but can be determined routinely using standard methods. A daily dose of about 0.01 to 500 mg/kg, preferably between about 0.01 and about 50 mg/kg, and more preferably about 0.01 and about 30 mg/kg body weight may be appropriate. The daily dose can be administered in one to four doses per day.

For therapeutic purposes, the medicaments of this invention are ordinarily combined with one or more adjuvants appropriate to the indicated route of administration. If administered per os, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose.

Formulations for parenteral administration may be in the form of aqueous or nonaqueous isotonic sterile injection solutions or suspensions. These solutions and suspensions may be prepared from sterile powders or granules using one or more of the carriers or diluents mentioned for use in the formulations for oral administration or by using other suitable dispersing or wetting agents and suspending agents. The compounds may be dissolved in water, Polyethylene glycol, propylene glycol, ethanol, corn oil, cottonseed oil, peanut oil, sesame oil, benzyl alcohol, sodium chloride, tragacanth gum, and/or various butters. Other adjuvants and modes of administration are well and widely known in the pharmaceutical art. The active ingredient may also be administered by injection as a composition with suitable carriers including saline, dextrose, or water, or with cyclodextrin (ie. Captisol), cosolvent solubilization (ie. propylene glycol) or micellar solubilization (ie. Tween 80).

The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed, including synthetic mono or diglycerides. In addition, fatty acids such as oleic acid find use in the preparation of injectables.

For pulmonary administration, the pharmaceutical composition may be administered in the form of an aerosol or with an inhaler including dry powder aerosol.

The pharmaceutical compositions may be subjected to conventional pharmaceutical operations such as sterilization and/or may contain conventional adjuvants, such as preservatives, stabilizers, wetting agents, emulsifiers, buffers etc. Tablets and pills can additionally be prepared with enteric coatings. Such compositions may also comprise adjuvants, such as wetting, sweetening, flavoring, and perfuming agents.

While specific dosing for antibodies in accordance with the invention has not yet been determined, antibody can be administered with weekly doses in the range of about 0.5 mg/kg to about 10 mg/kg, preferably about 2 mg/kg to about 3 mg/kg, or about 2 mg/kg. Antibody can be administered every two weeks with doses in the range of about I mg/kg to about 15 mg/kg, preferably about 3 mg/kg to about 10 mg/kg, or about 6 mg/kg. Antibody can be administered every three weeks with doses in the range of about 2 mg/kg to about 30 mg/kg, preferably about 5 mg/kg to about 15 mg/kg, or about 9 mg/kg. Some antibodies can be administered with doses in the range of 50 to 300 mg/m², where dosing in mg/m², as opposed to the conventional measurement of dose in mg/kg, is a measurement based on surface area. The therapeutically effective amount of EGFR antibody in the composition can be chosen from about 1 mg, about 2 mg, about 3 mg, about 4 mg, about 5 mg, about 6 mg, about 7 mg, about 8 mg, about 9 mg, about 10 mg, about 11 mg, about 12 mg, about 13 mg, about 14 mg, or about 15 mg.

Three distinct delivery approaches are expected to be useful for delivery of the antibodies in accordance with the invention. Conventional intravenous delivery, such as through a peripheral line or indwelling catheter over the length of time specified in the protocol, will presumably be the standard delivery technique for the majority of tumors. However, in connection with tumors in the peritoneal cavity, such as tumors of the ovaries, biliary duct, other ducts, and the like, intraperitoneal administration may prove favorable for obtaining high dose of antibody at the tumor and to minimize antibody clearance. In a similar manner certain solid tumors possess vasculature that is appropriate for regional perfusion. Regional perfusion will allow the obtention of a high dose of the antibody at the site of a tumor and will minimize short-term clearance of the antibody.

The antibody can be formulated in an aqueous buffer solution. The formulation may contain sodium chloride, sodium phosphate or sodium acetate at a physiological pH of about 5 to about 7.4. The formulation may or may not contain preservatives.

### Kits

The invention also describes kits comprising one or more EGFR antibody and one or more VEGF inhibitors in accordance with the foregoing. The inhibitors may be disposed in the kits in one or more containers. Each such container may contain separately or in admixture one or more EGFR antibody and one or more VEGF inhibitors in accordance with any of the foregoing. Typically, such kits are designed for medical use, and the inhibitors are comprised in pharmaceutically acceptable formulations. Among very highly preferred kits in this regard are those comprising panitumumab and AMG 706. Also among highly preferred embodiments in this regard are kits wherein the inhibitors are disposed in separate containers.

Further preferred kits are those that comprise integrally thereto or as one or more separate documents, information pertaining to the contents or the kit and the use of the inhibitors, Also among further preferred kits are those wherein the compositions are formulated for reconstitution in a diluent. In this regard, kits further comprising one or more containers of sterile diluent are especially preferred. Yet further preferred embodiments in this regard include kits wherein at least one of the inhibitors is disposed in vials under partial vacuum sealed by a septum and suitable for reconstitution to form a formulation effective for parental administration.

Preferred embodiments also include kits that provide single-dose packaging of one or more of the inhibitors. Preferred kits also include those that provide single and multi-chambered pre-filled syringes (e.g., liquid syringes and lyosyringes) for administering one or more of the inhibitors. Particularly preferred in this regard are kits in which the syringes are preloaded.

The invention will now be further described with reference to the following nonlimiting examples.

### EXAMPLE 1

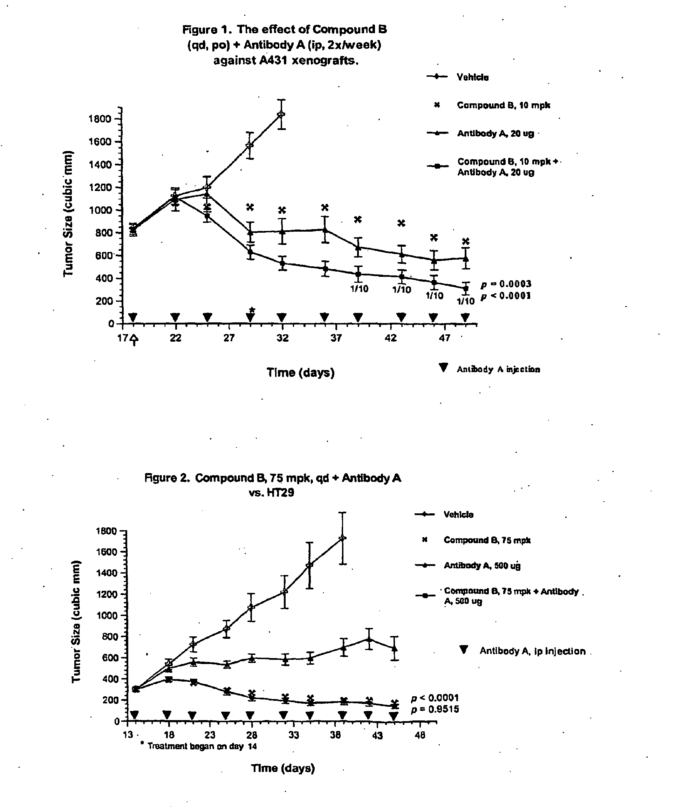

A431 epidermoid carcinoma cells (ATCC) were expanded in culture, harvested and injected subcutaneously into 5-8 week old female nude mice (CD1 nu/nu, Charles River Labs) (n = 5-15). Administration of VEGFR inhibitor B by oral gavage (10 mpk/dose) or by injection of anti-EGFR antibody A (20 ug/dose) or by a combination of VEGFR inhibitor B by oral gavage (10 mpk/dose) and by injection of anti-EGFR antibody A (20 ug/dose) began day 18 post tumor cell challenge. The VEGFR inhibitor was subsequently administered on a daily basis by oral gavage (10 mpk/dose) and the anti-EGFR antibody was administered injection (20 ug/dose) twice a week for the duration of the experiment. Progression of tumor growth was followed by three dimensional caliper measurements and recorded as a function of time. Initial statistical analysis was done by repeated measures analysis of variance (RMANOVA), followed by Scheffe post hoc testing for multiple comparisons. Vehicle alone (Ora-Plus, pH 2.0) or IgG2 injection (20 ug/dose) were the negative controls for the VEFGFR inhibitor and EGFR antibody, respectively. Substantial regression was noted for the combination therapy. Body weights were not negatively impacted by any treatment. Combination of VEGFR inhibitor B and anti-EGFR antibody A are most effective in the treatment of A431 cancer cells. See Figure 1. Body weights were not negatively impacted by any treatment.

### EXAMPLE 2

HT29 human colon carcinoma cells (ATCC) were expanded in culture, harvested and injected subcutaneously into 5-8 week old female nude mice (CD1 nu/nu, Charles River Labs) (n = 5-15). Administration of VEGFR inhibitor B by oral gavage (75 mpk/dose) or by injection of anti-EGFR antibody A (500 ug/dose) or by a combination of VEGFR inhibitor B by oral gavage (75 mpk/dose) and by injection of anti-EGFR antibody A (500 ug/dose) began day 14 post tumor cell challenge. The VEGFR inhibitor was subsequently administered on a daily basis by oral gavage (75 mpk/dose) and the anti-EGFR antibody was administered injection (500 ug/dose) twice a week for the duration of the experiment. Progression of tumor growth was followed by three dimensional caliper measurements and recorded as a function of time. Initial statistical analysis was done by repeated measures analysis of variance (RMANOVA), followed by Scheffe post hoc testing for multiple comparisons. Vehicle alone (Ora-Plus, pH 2.0) or IgG2 injection (500 ug/dose) were the negative controls for the VEFGFR inhibitor and EGFR antibody, respectively. Regression was noted for the combination therapy. See Figure 2. Combination of VEGFR inhibitor B and anti-EGFR antibody A is effective in the treatment of HT29 cancer cells.

### EXAMPLE 3

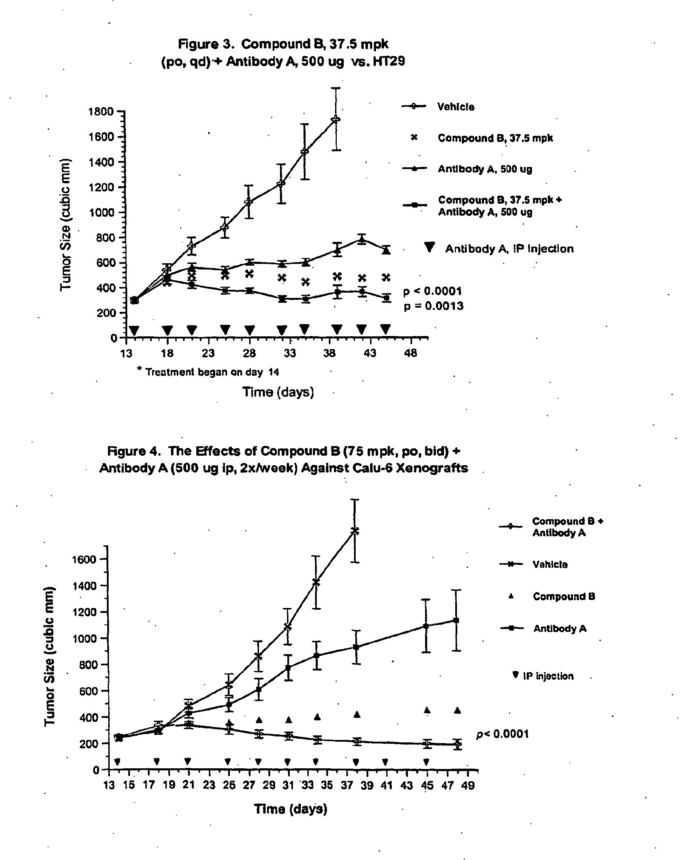

HT29 human colon carcinoma cells (ATCC) were expanded in culture, harvested and injected subcutaneously into 5-8 week old female nude mice (CD1 nu/nu, Charles River Labs) (n = 5-15). Administration of VEGFR inhibitor B by oral gavage (37.5 mpk/dose) or by injection of anti-EGFR antibody A (500 ug/dose) or by a combination of VEGFR inhibitor B by oral gavage (37.5 mpk/dose) and by injection of anti-EGFR antibody A (500 ug/dose) began day 14 post tumor cell challenge. The VEGFR inhibitor was subsequently administered on a daily basis by oral gavage (37.5 mpk/dose) and the anti-EGFR antibody was administered injection (500 ug/dose) twice a week for the duration of the experiment. Progression of tumor growth was followed by three dimensional caliper measurements and recorded as a function of time. Initial statistical analysis was done by repeated measures analysis of variance (RMANOVA), followed by Scheffe post hoc testing for multiple comparisons. Vehicle alone (Ora-Plus, pH 2.0) or IgG2 injection (500 ug/dose) were the negative controls for the VEFGFR inhibitor and EGFR antibody, respectively. Regression was noted for the combination therapy. See Figure 3. Combination of VEGFR inhibitor B and anti-EGFR antibody A are most effective in the treatment of HT29 cancer cells.

### EXAMPLE 4

CALU6 human non-small cell lung cancer cells (ATCC) were expanded in culture, harvested and injected subcutaneously into 5-8 week old female nude mice (CDI nu/nu. Charles River Labs) (n = 5-15). Administration of VEGFR inhibitor B by oral gavage (75 mpk/dose) or by injection of anti-EGFR antibody A (500 ug/dose) or by a combination of VEGFR inhibitor B by oral gavage (75 mpk/dose) and by injection of anti-EGFR antibody A (500 ug/dose) began day 14 post tumor cell challenge. The VEGFR inhibitor was subsequently administered on a daily basis by oral gavage (75 mpk/dose) and the anti-EGFR antibody was administered injection (500 ug/dose) twice a week for the duration of the experiment. Progression of tumor growth was followed by three dimensional caliper measurements and recorded as a function of time. Initial statistical analysis was done by repeated measures analysis of variance (RMANOVA), followed by Scheffe post hoc testing for multiple comparisons. Vehicle alone (Ora-Plus, pH 2.0) or IgG2 injection (500 ug/dose) were the negative controls for the VEFGFR inhibitors and EGFR antibody, respectively. Regression was noted for the combination therapy. See Figure 4. Combination of VEGER inhibitor B and anti-EGFR antibody A are most affective in the treatment of CALU6 cancer cells.

### EXAMPLE 5

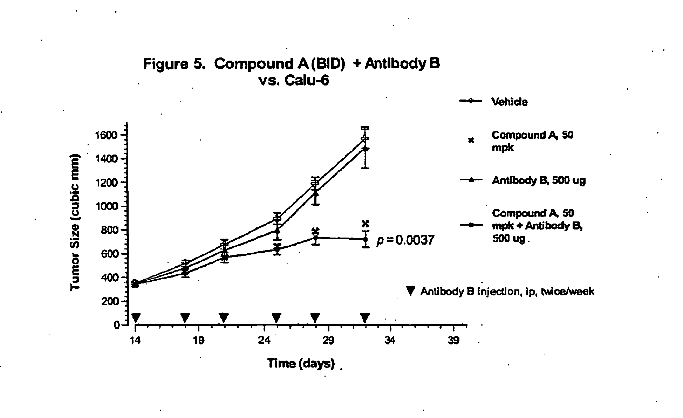

CALU6 human non-small cell lung cancer cells (ATCC) were expanded in culture, harvested and injected subcutaneously into 5-8 week old female nude mice (CDI nu/nu, Charles River Labs) (n = 5-15). Administration of VEGFR inhibitor A by oral gavage twice daily (50 mpk/dose) or by injection of EGFR antibody B (500 ug/dose) or by a combination of VEGFR inhibitor A by oral gavage twice daily (50 mpk/dose) and by injection of EGFR antibody B (500 ug/dose) began day 14 post tumor cell challenge. The VEGFR inhibitor was subsequently administered on a twice daily basis by oral gavage. (50 mpk(dose) and the aati-EGFR antibody was administered injection (500 ug/dose) twice a week for the duration of the experiment, Progression of tumor growth was followed by three dimensional caliper measurements and recorded as a function of time. Initial statistical analysis was done by repeated measures analysis of variance (RMANOVA), followed by Scheffe post hoc testing for multiple comparisons. Vehicle alone (Ora-Plus, pH 2.0) or IgG2 injection (500 ug/dose) were the negative controls for the VEFGFR inhibitor and EGFR antibody, respectively. Reduction of tumor size was noted for the combination therapy. See Figure 5. Combination of VEGFR inhibitor A and EGFR antibody B is effective in the treatment of CALU6 cancer cells.

No unacceptable toxological effects are expected when compounds of tho present invention are administered in accordance with the present invention.

## Claims

1. Use of an anti-EGFR fully human antibody in combination with a VEGFR inhibitor, wherein the anti-EGFR fully human antibody is panitumumab and the VEGFR inhibitor is N-(2,3-dihydro-3,3-dimethyl-1H-indol-6-yl)-2-[(4-pyridinylmethyl)amino]-3-pyridinecarboxamide, for manufacture of a medicament for the management or treatment of non-small cell lung cancer, colon carcinoma and epidermoid carcinoma in a subject.

2. The use of Claim 1, wherein the medicament is for the management or treatment of non-small cell lung cancer.

3. The use of Claim 1, wherein the medicament is for the management or treatment of colon out carcinoma.

4. The use of Claim 1, wherein the medicament is for the management or treatment of epidermoid carcinoma.

5. The use of any of Claims 1-4, wherein the anti-EGFR fully human antibody is administered in a dose of about 2mg/kg to about 3 mg/kg per week, about 5 mg/kg to about 7 mg/kg every two weeks or about 8 mg/kg to about 10 mg/kg every three weeks.

6. The use of any of Claims 1-4, wherein the VEGFR inhibitor is administered in a dose of about 25 mg to about 125 mg.

7. The use of any of Claims 1-4, wherein the VEGFR inhibitor is administered in a dose of about 75 mg twice a day.

8. The use of any of Claims 1-4, wherein the VEGFR inhibitor is administered in a dose of about 100 mg twice a day.

9. The use of any of Claims 1-4, wherein the VEGFR inhibitor is administered in a dose of about 125 mg once a day.

10. A combination of an anti-EGFR fully human antibody and a VEGFR inhibitor, wherein the anti-EGFR fully human antibody is panitumumab and the VEGFR inhibitor is N-(2,3-dihydro-3,3-dimethyl-1H-indol-6-yl)-2-[(4-pyridinylmethyl)amino]-3-pyridinecarboxamide, for use in the management or treatment of cancer in a subject, wherein the cancer is selected from non-small cell lung cancer, colon carcinoma and epidermoid carcinoma.

11. The combination of Claim 10, wherein the anti-EGFR fully human antibody is administered in a dose of about 2mg/kg to about 3 mg/kg per week, about 5 mg/kg to about 7 mg/kg every two weeks or about 8 mg/kg to about 10 mg/kg every three weeks.

12. The combination of Claim 10, wherein the VEGFR inhibitor is administered in a dose of about 25 mg to about 125 mg.

13. The combination of Claim 10, wherein the VEGFR inhibitor is administered in a dose of about 75 mg twice a day.

14. The combination of Claim 10, wherein the VEGFR inhibitor is administered in a dose of about 100 mg twice a day.

15. The combination of Claim 10, wherein the VEGFR inhibitor is administered in a dose of about 125 mg once a day.

## Patentansprüche

1. Verwendung eines vollständig menschlichen anti-EGFR-Antikörpers in Kombination mit einem VEGFR-Inhibitor, wobei es sich bei dem vollständig menschlichen anti-EGFR-Antikörper um Panitumumab und bei dem VEGFR-Inhibitor um N-(2,3-Dihydro-3,3-dimethyl-1H-indol-6-yl)-2-[(4-pyridinylmethyl)amino]-3-pyridincarboxamid handelt, zur Herstellung eines Medikaments für das Management oder die Behandlung von nicht kleinzelligem Lungenkrebs, von Colonkarzinom und von Epidermoidkarzinom bei einem Probanden.

2. Verwendung nach Anspruch 1, wobei das Medikament für das Management oder die Behandlung von nicht kleinzelligem Lungenkrebs vorgesehen ist.

3. Verwendung nach Anspruch 1, wobei das Medikament für das Management oder die Behandlung von Colonkarzinom vorgesehen ist.

4. Verwendung nach Anspruch 1, wobei das Medikament für das Management oder die Behandlung von Epidermoidkarzinom vorgesehen ist.

5. Verwendung nach einem der Ansprüche 1-4, wobei der vollständig menschliche anti-EGFR-Antikörper in einer Dosis von etwa 2 mg/kg bis etwa 3 mg/kg pro Woche, von etwa 5 mg/kg bis etwa 7 mg/kg alle zwei Wochen oder von etwa 8 mg/kg bis etwa 10 mg/kg alle drei Wochen verabreicht wird.

6. Verwendung nach einem der Ansprüche 1-4, wobei der VEGFR-Inhibitor in einer Dosis von etwa 25 mg bis etwa 125 mg verabreicht wird.

7. Verwendung nach einem der Ansprüche 1-4, wobei der VEGFR-Inhibitor in einer Dosis von etwa 75 mg zweimal pro Tag verabreicht wird.

8. Verwendung nach einem der Ansprüche 1-4, wobei der VEGFR-Inhibitor in einer Dosis von etwa 100 mg zweimal pro Tag verabreicht wird.

9. Verwendung nach einem der Ansprüche 1-4, wobei der VEGFR-Inhibitor in einer Dosis von etwa 125 mg einmal pro Tag verabreicht wird.

10. Kombination eines vollständig menschlichen anti-EGFR-Antikörpers und eines VEGFR-Inhibitors, wobei es sich bei dem vollständig menschlichen anti-EGFR-Antikörper um Panitumumab und bei dem VEGFR-Inhibitor um N-(2,3-Dihydro-3,3-dimethyl-1H-indol-6-yl)-2-[(4-pyridinylmethyl)amino]-3-pyridincarboxamid handelt, zur Verwendung bei dem Management oder der Behandlung von Krebs bei einem Probanden, wobei der Krebs aus nicht kleinzelligem Lungenkrebs, Colonkarzinom und Epidermoidkarzinom ausgewählt ist.

11. Kombination nach Anspruch 10, wobei der vollständig menschliche anti-EGFR-Antikörper in einer Dosis von etwa 2 mg/kg bis etwa 3 mg/kg pro Woche, von etwa 5 mg/kg bis etwa 7 mg/kg alle zwei Wochen oder von etwa 8 mg/kg bis etwa 10 mg/kg alle drei Wochen verabreicht wird..

12. Kombination nach Anspruch 10, wobei der VEGFR-Inhibitor in einer Dosis von etwa 25 mg bis etwa 125 mg verabreicht wird.

13. Kombination nach Anspruch 10, wobei der VEGFR-Inhibitor in einer Dosis von etwa 75 mg zweimal pro Tag verabreicht wird.

14. Kombination nach Anspruch 10, wobei der VEGFR-Inhibitor in einer Dosis von etwa 100 mg zweimal pro Tag verabreicht wird.

15. Kombination nach Anspruch 10, wobei der VEGFR-Inhibitor in einer Dosis von etwa 125 mg einmal pro Tag verabreicht wird.

## Revendications

1. Utilisation d'un anticorps entièrement humain anti-EGFR, en combinaison avec un inhibiteur du VEGFR, l'anticorps entièrement humain anti-EGFR étant panitumumab et l'inhibiteur du VEGFR étant N-(2,3-dihydro-3,3-diméthyl-1H-indol-6-yl)-2-[(4-pyridinylméthyl)amino]-3-pyridinecarboxarade, pour la fabrication d'un médicament destiné à la prise en charge ou au traitement d'un cancer du poumon non à petites cellules, d'un carcinome du côlon et d'un carcinome épidermoïde chez un sujet.

2. Utilisation selon la revendication 1, dans laquelle le médicament est destiné à la prise en charge ou au traitement d'un cancer du poumon non à petites cellules.

3. Utilisation selon la revendication 1, dans laquelle le médicament est destiné à la prise en charge ou au traitement d'un carcinome du côlon.

4. Utilisation selon la revendication 1, dans laquelle le médicament est destiné à la prise en charge ou au traitement d'un carcinome épidermoïde.

5. Utilisation selon l'une quelconque des revendications 1 à 4, dans laquelle l'anticorps entièrement humain anti-EGFR est administré à une dose de 2 mg/kg environ à 3 mg/kg environ par semaine, de 5 mg/kg environ à 7 mg/kg environ toutes les deux semaines ou de 8 mg/kg environ à 10 mg/kg environ toutes les trois semaines.

6. Utilisation selon l'une quelconque des revendications 1 à 4, dans laquelle l'inhibiteur du VEGFR est administré à une dose de 25 mg environ à 125 mg environ.

7. Utilisation selon l'une quelconque des revendications 1 à 4, dans laquelle l'inhibiteur du VEGFR est administré à une dose de 75 mg environ deux fois par jour.

8. Utilisation selon l'une quelconque des revendications 1 à 4, dans laquelle l'inhibiteur du VEGFR est administré à une dose de 100 mg environ deux fois par jour.

9. Utilisation selon l'une quelconque des revendications 1 à 4, dans laquelle l'inhibiteur du VEGFR est administré à une dose de 125 mg environ une fois par jour.

10. Combinaison d'un anticorps entièrement humain anti-EGFR et d'un inhibiteur du VEGFR, l'anticorps entièrement humain anti-EGFR étant panitumumab et l'inhibiteur du VEGFR étant N-(2,3-dihydro-3,3-diméthyl-1H-indol-6-yl)-2-[(4-pyridinyl-méthyl)-amino]-3-pyridinecarboxamide, pour l'utilisation dans la prise en charge ou le traitement d'un cancer chez un sujet, le cancer étant choisi parmi un cancer du poumon non à petites cellules, un carcinome du côlon et un carcinome épidermoïde.

11. Combinaison selon la revendication 10, dans laquelle l'anticorps entièrement humain anti-EGFR est administré à une dose de 2 mg/kg environ à 3 mg/kg environ par semaine, de 5 mg/kg environ à 7 mg/kg environ toutes les deux semaines ou de 8 mg/kg environ à 10 mg/kg environ toutes les trois semaines.

12. Combinaison selon la revendication 10, dans laquelle l'inhibiteur du VEGFR est administré à une dose de 25 mg environ à 125 mg environ.

13. Combinaison selon la revendication 10, dans laquelle l'inhibiteur du VEGFR est administré à une dose de 75 mg environ deux fois par jour.

14. Combinaison selon la revendication 10, dans laquelle l'inhibiteur du VEGFR est administré à une dose de 100 mg environ deux fois par jour.

15. Combinaison selon la revendication 10, dans laquelle l'inhibiteur du VEGFR est administré à une dose de 125 mg environ une fois par jour.
